# EUROPEAN PATENT APPLICATION

(11) **EP 1 431 306 A1**
(43) Date of publication of application: **23.06.2004**
(21) Application number: 02028510.2
(22) Date of filing: 19.12.2002
(51) Int. Cl.: C07K 14/16, G01N 33/68, A61K 38/16, A61P 31/18

(54) **A mechanism for hiv-1 entry into host cells and peptides inhibiting this mechanism**

(71) Applicant: Creabilis Therapeutics s.r.l., 10010 Colleretto Giacosa (TO) (IT)
(72) Inventor: Bussolino, Frederico, 10129 Torino (IT); Marchio, Serena, 10134 Torino (IT)
(74) Representative: Weiss, Wolfgang, Dipl.-Chem. Dr.

(57) **Abstract**

This invention relates to the finding that TAT interacts with gp120 at the cell surface and enhances HIV-1 entry into host cells. This invention also discloses modulators of this interaction, particularly peptides that mimic the region of gp120 involved in this interaction. These peptides interfere with TAT-mediated enhancement of infection regardless of virus strain, and are therefore suitable for the development in general of broad-range drugs against AIDS and other infectious diseases induced by HIV-1 related pathogens, and specifically for a class of chemicals active in the reduction or abrogation of virus and infectivity spreading.

## Description

### Field of the invention

This invention relates to the finding of a new mechanism of HIV-1 infection. This invention further relates to the development of novel drugs, e.g. synthetic peptide drugs blocking this mechanism, therefore containing HIV-1 infection.

### Background of the invention

Several drugs have been developed to manage Human Immunodeficiency Virus-1 (HIV-1) infection and the consequent onset of AIDS. The advent of highly active antiretroviral therapy (HAART) ― combinations of protease and reverse transcriptase inhibitors - provided a potent and clinically effective method of suppressing viral load in HIV-1-infected individuals. However, although initially successful, a broader clinical experience has revealed limitations in this therapeutic regimen. Consistent and long-term clinical benefit from triple therapy remains elusive owing to intolerance, non-compliance with drug dosing schedules and, most ominously, the development and transmission of resistant viruses. As a consequence, the search for anti-HIV agents continues, with renewed emphasis placed on novel targets in an attempt to increase the repertoire and efficacy of clinically available drugs.

Although the HIV-1 entry process was one of the earliest mechanisms examined as a target for therapeutic intervention, progress was difficult and success elusive. However, the elucidation of co-receptors that facilitate virus entry by triggering fusion, and the recently solved X-ray crystal structure of elements of the envelope glycoprotein gp120, have provided crucial new insights into the mechanism of HIV-1 entry and afforded significant new opportunities for HIV-1 drug discovery.

Virion attachment is mediated by the specific binding of gp120 to the receptor CD4, although it is also facilitated by non-specific interactions with other cell-surface molecules. The interaction of gp120 with CD4 results in the exposure of domains of gp120 that subsequently interact with one of several cell type-specific co-receptors, leading to destabilization of the gp 120-gp41 protein complex. As a consequence, the gp41 subunit undergoes a conformational rearrangement, exposing the hydrophobic fusion peptide that inserts into the host cell membrane and initiates the fusion process (Chan and Kim, Cell 93 (1998), 681-684; Wyatt and Sodroski, Science 280 (1998), 1884-1888). This process occurs not only at the first interaction between the host cells and the virus, but characterizes the succeeding waves of spreading from lymphonodes, where HIV-1 replicates after the first infection, to the circulating lymphocytes. HIV-1 entry has been validated as a clinically relevant target, as demonstrated by several compounds that have been advanced into clinical trials (Table 1) (Blair et al., Drug Discov. Today 5 (2000), 183-194).

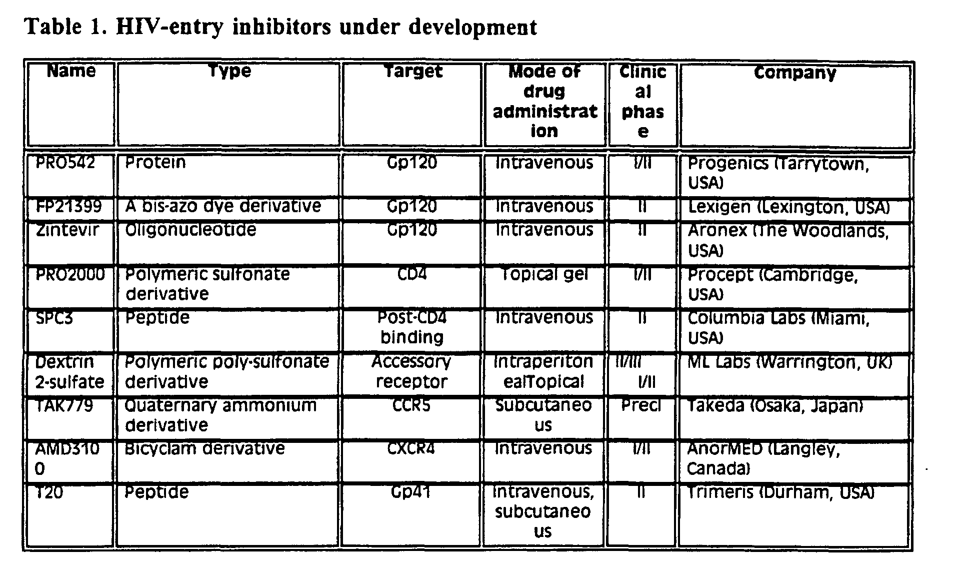

These reports confirm that discovering new targets is a crucial step for the development of innovative drugs to block virus entry. To be more efficient, these new drugs must be specific, but of broad-range application, that is, they have to block infection regardless of the viral strain. This characteristic is mandatory to fully circumvent drug resistance.

Recent attention has been placed in investigating the multifaceted activities of HIV-1 Trans Activator of Transcription (TAT) (Jeang et al., J. Biol. Chem. 274 (1999), 28837-28840). TAT is a nuclear factor with the main function of enhancing the transcription of viral RNAs, thus allowing the production of new viral particles and the consequent spreading of the virus.

Several other TAT functions have been described, and somehow related to AIDS pathogenesis. In particular, TAT is secreted by infected cells and is therefore detectable in the extracellular milieu, where acts as a growth factor binding trans-membrane receptors and inducing cellular pathways (Albini et al., Nat. Med. 2 (1996), 1371-1375); Mitolo et al., Blood 90 (1997), 1365-1372), or enters surrounding cells interfering with their gene expression. Interestingly, after release a portion of the protein binds the extracellular matrix (ECM)-associated heparan sulphate proteoglycans (HSPG) (Chang et al., Aids 11 (1997), 1421-1431). So, TAT is partially sequestered at the cell surface, which is, as previously described, the site for HIV-1 recognition and entry.

In the past few years, some peculiarities of TAT have been applied to biotechnological uses. In particular, a basic peptide derived from TAT (positions 48-60) has been reported to have the ability to translocate through the cell membranes and accumulate in the nucleus, and has been employed for the delivery of exogenous proteins into the cells (Morris et al., Nat. Biotechnol. 19 (2001), 1173-1176). Moreover, TAT peptides facilitate intracellular delivery also of small colloidal particles, and of relatively large drug carriers, such as 200-nm liposomes (Torchilin et al., PNAS USA 98 (2001), 8786-8791). The mature HIV-1 particle has a diameter of about 100 nm, suggesting that such a mechanism of internalisation could be possible also *in vivo.*

The contribution of extracellular TAT to the progression of viral infection has been underlined by the ability of neutralizing anti TAT antibodies to reduce the viral load both *in vitro* and *in vivo* (Cafaro et al., Nat. Med. 5 (1999), 643-650; Goldstein, Nat. Med. 2 (1996), 960-964). These reports suggest that extracellular TAT can play a functional role in HIV-1 infection. However, until now the presence of TAT on the cell membranes and the ability of TAT basic region to deliver big particles into the cells have never been related, nor consequently investigated.

### Brief description of the drawings

- **Fig. 1**: A is the map of the retroviral vector PINCO, where TAT coding sequence was inserted in the BamHI/EcoRI cloning site; B is the Northern Blot analysis showing the expression of TAT mRNA in U937/PINCO and U937/TAT cells.
- **Fig. 2**: A and C are the anti-TAT surface immunostainings; B is the graph of the CAT assay, where the line is the calibration curve done with rTAT and bars are the activities of the pool (U937/TAT) and the clones (Clone 1, 2 and 3) of TAT-expressing U937 cells.
- Fig. 3: are the entry experiments performed with lentiviral vectors on C8166 cells. A is the effect of the co-culture with U937/TAT cells on TAT+/gp120-LV entry; B is the effect of rTAT; C is the time-course and D is the dose-response effect of the co-culture with U937/TAT cells on the entry of VSV-G-LV compared to TAT + /gp 120-LV.
- **Fig. 4**: shows in **A** the BiaCORE analysis of gp120/TAT binding and in **B** the GST-TAT mutants binding to gp120 (left panel) and effect on enhancement of TAT-/gp120-LV entry into C8166 celss (right panel).
- **Fig. 5**: is the proposed model of interaction between gp120 and TAT at the cell surface.
- **Fig. 6**: is the binding of the selected phage to rTAT-coated microwells.
- **Fig. 7**: is the percent variation in TAT-/gp120-LV basal entry into C8166 in the presence of 100 µM of the peptides.
- **Fig. 8**: is the percent variation in TAT + /gp 120-LV basal entry into C8166 in the presence of 100 µM of the peptides.
- **Fig. 9**: is the percent variation in TAT-/gp 1 20-LV entry into C8166 in the presence of 100 µM of the peptides, after co-culture with U937/PINCO or U937/TAT cells.
- **Fig. 10**: is the percent variation in TAT+/gp120-LV entry into C8166 in the presence of 100 µM of the peptides, after co-culture with U937/PINCO or U937/TAT cells.
- **Fig. 11**: is the percent variation in TAT+/gp120-LV entry into C8166 cells in the presence of protease inhibitors, after co-culture with U937/TAT cells.
- **Fig. 12**: is the infection of PBMC with HIV-1 strain III B and Ba-L. A is the effect of the peptides on the basal infection; B is the effect of the peptides on the infection following addition of 7 nM rTAT.

### Summary of the invention

In accordance with one aspect of the present invention, the inventors have found a novel mechanism for HIV-1 entry into host cells. In particular, they have shown that HIV-1 TAT is sequestered at the cell surface, where it acts as a novel, specific receptor for gp120. This interaction enhances virus entry into permissive cells. This mechanism is independent of the viral strain, having similar outcomes on the infection by M-tropic (Ba-L) and L-tropic (III B) viruses. The inventors have further suggested a simple model of pathogenesis: HIV-1-infected cells release TAT into their environment; as a consequence, TAT binds the surface of surrounding, still uninfected cells, thus rendering those cells more permissive to HIV-1 infection.

Consequently, inhibitors of the interaction between HIV-1 TAT protein and HIV-1 gp120 are capable of inhibiting the entry of HIV-1 into a host cell. These inhibitors may be used for diagnostic or therapeutic applications, particularly for the treatment of HIV-1 infections. Further, methods and systems, e.g. kits, for identifying novel inhibitors are provided.

Second, the inventors have mapped the region of gp120 involved in TAT binding, by screening Phage Display libraries on TAT-expressing cells. In particular, two peptides were selected as specific TAT ligands, identifying a portion of the gp120 V1/V2 loop. The inventors have shown that these peptides, and they derivatives, interfere with TAT/gp120 interaction and revert TAT-mediated enhancement of virus entry. Finally, the inventors have shown that the peptides are equally efficient in inhibiting the infection of both M-tropic (Ba-L) and L-tropic (III B) HIV-1 strains.

Thus, the invention further relates to novel specific TAT ligands, particularly peptides selected from the gp 120 V1/V2 loop or peptides homologous thereto.

### Detailed Description of the invention

A first aspect of the present invention relates to the use of inhibitors of the interaction between the HIV-1 TAT protein and HIV-1 gp120 for inhibiting the entry of HIV-1 into a host cell, particularly a human host cell.

In a first embodiment of this aspect, the inhibitor is selected from compounds which are capable of binding to TAT, more particularly from compounds which are capable of competing with gp120 for the binding to TAT. In a further embodiment, the inhibitor is selected from compounds which are capable of binding to gp120, particularly from compounds which compete with TAT for the binding to gp120.

The inhibitors may be a peptide, e.g. a peptide having a length of from 4 to 25, particularly from 5 to 20 and more particularly from 6 to 15 amino acids including cyclic peptides, peptides containing non-naturally occuring amino acids, e.g. D-amino acids or peptide mimetics. In an especially preferred embodiment the peptide is homologous to the gp120 V1/V2 region. On the other hand, the inhibitor may be a non-peptidic compound, e.g. a low-molecular weight organic compound, particularly having a molecular weight up to 2000 Da. In an other embodiment, the peptide mimics the region of TAT involved in the interaction with gp120.

Specific examples of preferred peptidic inhibitors are selected from:
(a) CTVECYFNCTPTC (SEQ ID No. 2)
(b) CPDRKKKVVMVC (SEQ ID No. 3)
(c) CSFNITTTEIRDKVKK (SEQ ID No. 127)
(d) a peptide comprising at least 5 contiguous amino acids from a peptide, selected from the group consisting of peptides (a) - (c),
(e) a peptide which has a sequence identity of at least 80 % to the amino acid sequence of a peptide selected from the group consisting of peptides (a) - (d).

Further specific examples of preferred peptidic inhibitors are selected from:
(a) RDKKKK (SEQ ID No. 40),
(b) RDKKKQ (SEQ ID No. 41),
(c) RDKKKV (SEQ ID No. 42),
(d) RNKRKQ (SEQ ID No. 51),
(e) RDKTQK (SEQ ID No. 52),
(f) DRKKKV (SEQ ID No. 43),
(g) KDKKEK (SEQ ID No. 45),
(h) RDKQQK (SEQ ID No. 49),
(i) RDKVQK (SEQ ID No. 50),
(j) CSFNIT (SEQ ID No. 4),
(k) RDKVKK (SEQ ID No. 44),
(l) a peptide comprising at least 5 contiguous amino acids from a peptide selected from the group consisiting of peptides (a) -(k),
(m) a peptide which has a squence identity of at least 80 % to the amino acid sequence of peptide selected from the group consisting of peptides (a) - (l).

The inhibitors of the invention may be used for research purposes, e.g. in order to study the mechanism of HIV-1 infection. Further, the inhibitors are suitable for diagnostic and therapeutic applications.

In a preferred embodiment, the inhibitors are used for the manufacture of medicament for the treatment of HIV-1 infection, particularly for the containment of HIV-1 infection. Surprisingly, the inhibitors are capable for the treatment of infection caused by different HIV-1 strains, particularly M-tropic and L-tropic HIV-1 strains.

Thus a further aspect of the present invention relates to a pharmaceutical composition comprising as an active ingredient at least one inhibitor of the interaction between HIV-1 TAT protein and HIV gp120 and optionally pharmaceutically acceptable carriers, diluents and adjuvants. The composition may be in the form of a solution, suspension, emulsion, tablet, dragee, capsule, cream, ointment etc. The manufacture of the composition usually comprises a therapeutically effective amount of the active ingredient with pharmaceutically acceptable carriers, diluents and/or adjuvants according to known formulation methods. The composition may be administered by any suitable route, e.g. by parenteral, oral, topical, mucosal, or nasal administration.

The therapeutic effective dose of the ingredient may be determined by a skilled person without undue burden. Generally, e.g. for peptidic compounds, their therapeutically effective doses are in the range of 10 to 200 mg daily.

Still a further aspect of the present invention relates to a method for identifying and/or characterizing a compound which inhibits the entry of HIV-1 into a host cell comprising
(i) providing at least one compound to be tested and
(ii) determining if the compound is capable of inhibiting the interaction between HIV-1 TAT protein and HIV-1 gp120.

In one embodiment the method is a screening method, wherein a plurality of compounds is tested in parallel or sequencial. For example, a compound library, e.g. a phage display library of peptidic compounds, a chemical library etc. may be tested.

Alternatively, the method is suitable for characterizing the property of an already known inhibitor, which may be used as a lead structure from which derivatives may be obtained, e.g. by molecular modelling and/or random variations.

The method may be carried out as a cellular-based assay, e.g. an assay wherein the inhibition of the infection of host cells by HIV viruses or HIV analogues is determined in the presence of the compound to be tested, optionally in comparision to a suitable control. On the other hand, the method may comprise a molecular-based assay, wherein the influence of a test compound on the interaction between purified e.g. recombinant TAT and gp120 is determined.

Finally, the method encompasses the formulation of a compound which has been identified as an inhibitor by the method as described above or a compound derived therefrom as a pharmaceutical composition.

The inhibitor of the present invention may be administered alone. Preferably, however, the inhibitor is administered as a component in a combination of different anti HIV agents, e.g. protease and/or reverse transcriptase inhibitors. On the basis of the mechanism of action of this type of inhibitors and on the proposed model of pathogenesis, this class of molecules can be characterized by anti-spreading activity.

### Experimental section

1. The first part of this section relates to the finding that TAT is released by producing cells and binds the cell surfaces, where it acts as an additional co-receptor for virus entry. In particular, in this part we will describe how the experimental model was designed and which are its properties, how TAT interacts with gp120 and enhances virus entry. Finally, we will propose a model of TAT-driven pathogenesis for the spreading of HIV-1.

### 1.1 Description of the experimental model

We have set up a model aimed to mimic *in vitro* the production of TAT by infected cells, observed *in vivo* in patients with AIDS (Westendorp et al., Nature 375 (1995), 497-500). This model consists of U937 pro-monocyte cells expressing TAT₈₆ of HIV-1 strain III B (SEQ ID NO.1). In this paragraph we describe how the cells were prepared and characterized.

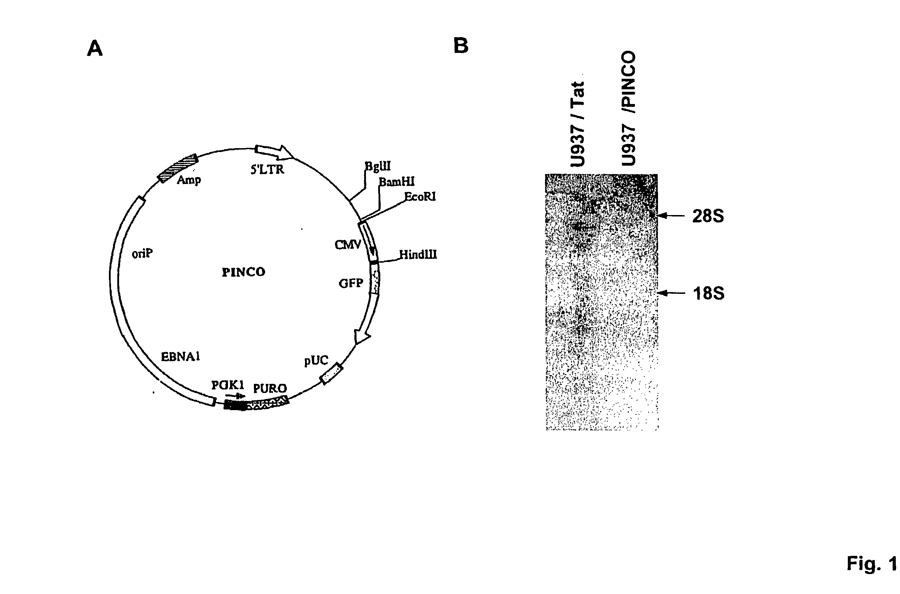

Fig. 1 A shows the map of the retroviral construct PINCO (Grignani et al., Cancer Res. 58 (1998), 14-19), in which we have inserted the coding sequence of TAT into the BamHI/EcoRI cloning site, producing the PINCO/TAT vector. We have transduced U937 cells with PINCO/TAT and named these cells U937/TAT. Cells mock-transduced are named U937/PINCO and used as a negative control. Fig. 1 B shows the Northern Blot analysis of TAT expression in U937/TAT cells. Since PINCO allows also the expression of a reporter gene, the *Green Fluorescent Protein* (GFP), the transduced cells can be easily purified from the population. By this means, we have isolated different clones (by limited dilution) and pools (by cell sorting with a FACS sorter) of U937/TAT cells.

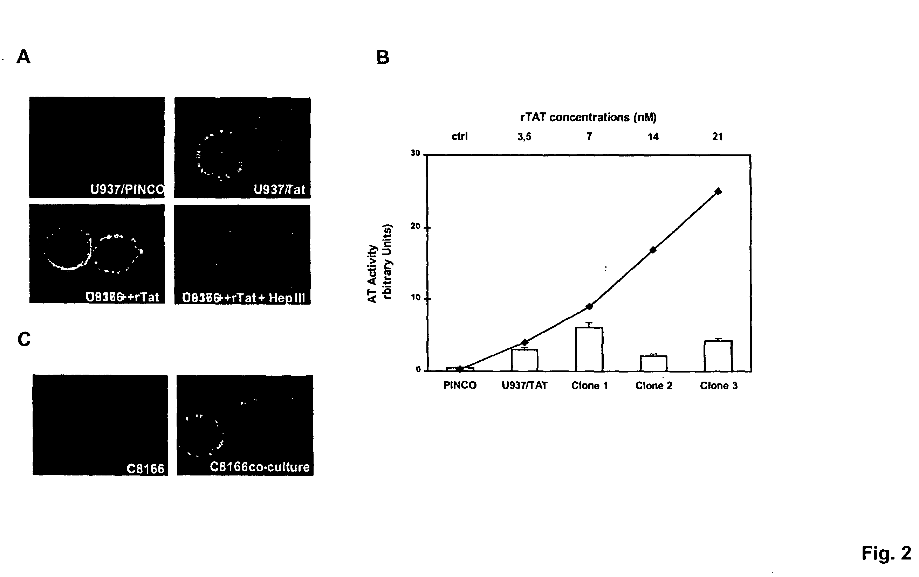

To characterize the U937/TAT cells, we have first evaluated the presence of TAT on their membranes. Fig. 2A demonstrates that the NT3 2 D1 anti-TAT mAb specifically stains the surfaces of U937/TAT cells. The same figure also shows that recombinant TAT (rTAT), when added to the culture medium of U937 cells at a final concentration of 7 nM, localizes at the cell membranes. If the cells are treated with the enzyme Heparanase III, sulphate groups are detached from HSPG and TAT is no more able to bind the cell surfaces.

We have then demonstrated that TAT is present in the conditioned media of U937/TAT cells. Since anti-TAT antibodies are poorly efficient, we could not detect TAT protein by Western Blot, suggesting that it is present in low amounts. To circumvent this problem we have tested the presence of TAT trans-activating activity in the culture media by a conventional CAT assay. The graph in Fig. 2B demonstrates the presence of TAT in U937/TAT cell media. By comparison with a standard curve done with rTAT, we have quantified the amount of released TAT in a concentration range of 1,5-20 pmol/10⁶ cells.

To complete the model, we have finally evaluated if TAT can bind the surface of close, untransfected cells. This simplified model should reproduce the way in which infected cells interact *via* TAT with surrounding, still uninfected cells. We have set up a co-culture system, in which U937/TAT cells are cultured in the lower compartment and C8166 T-lymphocyte cells are placed in the upper compartment of a transwell with a pore size of 0,4 µM (Falcon). The two compartments are divided by a membrane that allows the exchange of secreted proteins but not of the cells themselves. Fig. 2C shows that an anti-TAT surface staining is present on C8166 cells maintained in co-culture with U937/TAT cells for 4 h.

### 1.2 Role of cell membrane-bound TAT in HIV-1 entry into the cells.

To assess the role of surface-bound TAT in HIV-1 infection, we have performed entry experiments using HIV-1-derived lentiviral vectors (Dull et al., J. Virol 72 (1998), 8463-8471; Naldini et al., PNAS USA 93 (1996), 11382-11388). These vectors are a modification of the wild type virus, in order to reach higher safety for the operator, and easier detection of the infected cells. A complete viral particle is assembled by transfecting packaging cells (the human renal cancer cell line 293T) with 3 vectors. The first one carries all the genes of HIV-1 but the *env* gene. The second one carries the gene for the envelope protein (thus allowing the construction of viruses pseudotyped with different envelopes). The third one is a reporter vector for the expression of GFP. The mechanism of entry is the same as for the wild type virus, with the difference that transduced cells express GFP and can be readily detected by FACS analysis after 72 h from the infection. Moreover, since transduced cells are unable to produce new virions and to propagate the infection, by using these constructs it is possible to dissect the infection process, and to evaluate exclusively the phase of virus entry.

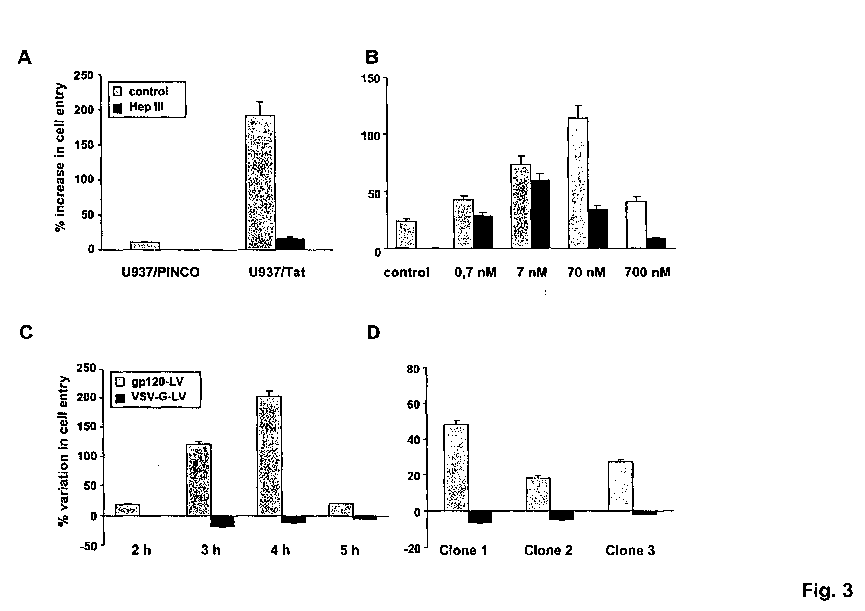

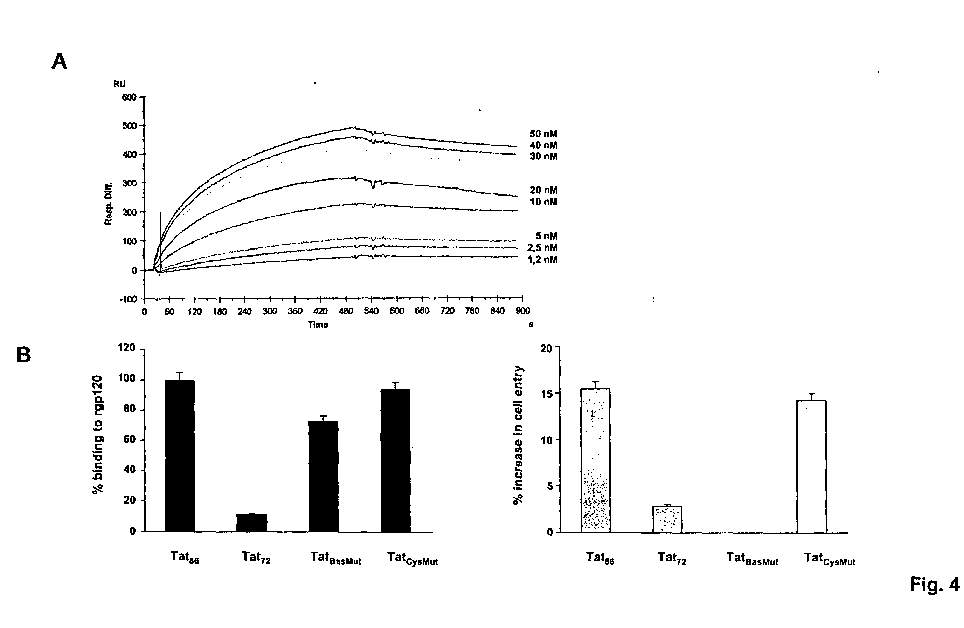

Fig. 3A shows that C8166 cells maintained in co-culture with U937/TAT cells for 4 h are up to three times more permissive to the entry of lentiviral vectors carrying the gp120 envelope of HIV-1 HXB2 (TAT+/gp120-LV). This effect is reverted if the cells are pre-treated with Heparanase III. To exclude interferences due to factors eventually regulated by TAT itself in U937/TAT cells, we have also pre-incubated C8166 cells with rTAT for 15 min, observing a dose-response effect in increasing TAT + /gp 120-LV entry. Again, this effect is reverted by pre-treating the cells with Heparanase III (Fig. 3B). To investigate the specificity of this effect, we have used lentiviral vectors pseudotyped with a different envelope, i.e. Vescicular Stomatitis Virus-G (VSV-G-LV), to transduce C8166 cells after co-culture with U937/TAT cells for different incubation times, or with the different U937/TAT clones for 2 h. Fig. 3C and D show that, independently from the incubation time and from the amount of TAT, VSV-G-LV entry is not enhanced following the co-culture. Therefore, the increase in cell entry is strictly dependent not only on surface-bound TAT, but also on the presence of gp120. We have hypothesized that a direct interaction between TAT and gp120 might be the mechanism of TAT-dependent enhancement of TAT+/gp120-LV entry into the cells. This hypothesis is demonstrated in Fig. 4A, where a surface-plasmon resonance analysis is shown. rTAT immobilized at a BIAcore sensor chip specifically binds the recombinant gp 120 III B protein (rgp 120) with a Kd = 8 ± 2 nM.

### 1.3 Discovering of the portion of TAT that binds to gp120

To determine the region of TAT required to bind gp120 we have produced different TAT from HIV-1 HXB2 as GST-fusion proteins, including full-length TAT (TAT₈₆) and mutants lacking the C-terminal (TAT₇₂) or containing mutated basic (TAT_{BasMut}) or cystein-rich (TAT_{CysMut}) domains, and assayed the ability of these fusion proteins to bind gp120. All variants except for TAT₇₂ could bind rgp 120-coated microwells, suggesting that the interaction with gp120 is mediated by TAT aminoacids 72-86 (Fig. 4B, left panel). To confirm this finding, we have incubated C8166 cells with 7µM of each GST-TAT variant for 15 min before incubation with TAT/gp 120-LV and have found that only TAT_{CysMut} could enhance vector entry into the cells compared to wild-type TAT₈₆ (Fig. 4B, right panel). These results demonstrate that both TAT C-terminal and basic domains are crucial for the enhancement of TAT/gp 120-LV entry. It is not surprising that TAT_{BasMut}, although retaining the ability to bind gp120, was ineffective in enhancing virus entry, since this protein cannot bind heparan sulphate proteoglycans and is consequently not sequestered at the cell surface.

### 1.4 Proposed model of pathogenesis

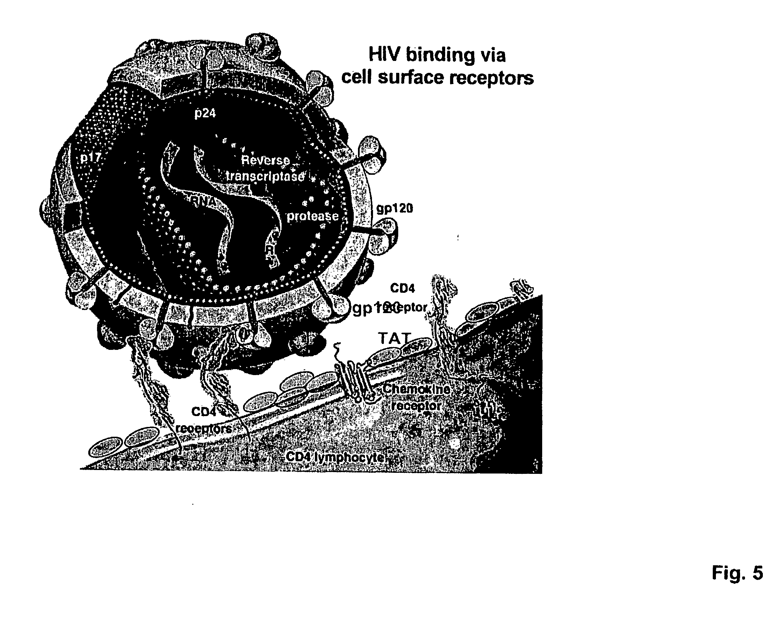

We propose the following mechanism (see drawing in Fig. 5):
HIV-1-infected cells release TAT into their environment; as a consequence, TAT binds the surface of surrounding, still uninfected cell, thus rendering those cells more permissive to HIV-1 infection. This amplification possibly causes an acceleration in the spreading of the virus. Until now the knowledge of TAT functions was not sufficient to confirm its direct involvement in HIV-1 infection. We demonstrate for the first time that these events depend on an enhanced TAT-driven virus entry into the cells, due to a specific interaction among gp 120 and TAT at the cell surface.

This mechanism suggest a new target for the development of anti-HIV-1 drugs. In fact, blocking the TAT/gp120 interaction should inhibit the virus infection due to membrane-bound TAT. In the following part the study of such drugs is described in details.

**2.** The second part of this section relates to the discovery of peptides blocking the entry mechanism described in the first part. The described peptides have been selected from a random Phage Display library, and share homology with a region of gp120 whose function was unknown until now. We will describe how the peptide sequences have been selected and characterized. Moreover, applications of the cognate synthetic peptides as anti-HIV-1 drugs are disclosed.

### 2.1 Development of targeted drugs by Phage Display screening

In a Phage Display library, a random oligonucleotide is inserted immediately before the gene coding for the pIII coat protein so that, when the phage particle is assembled, a random peptide appears on its surface as a fusion with the pIII protein itself. In a library, up to 10⁹ different peptides are displayed on the phage surface, from which it is possible to select one or more peptides specific for binding a substrate of interest (Scott and Smith, Science 249 (1990), 386-390). The peptide drugs described in this invention have been developed by screening Phage Display libraries on U937/TAT cells. Accordingly to the model described in Part 1, one of the most representative proteins on the surface of U937/TAT cells should be TAT itself. Two different libraries, CX₁₀C and CX₃CX₃CX₃C, where C is Cys and X is any amino acid, have been used. After the second (CX₃CX₃CX₃C library) or third round (CX₁₀C library) of selection, we have obtained a significant enrichment in phage binding to U937/TAT cells compared to U937/PINCO cells. Sequencing the selected phage has revealed an enrichment in two peptides (phage inserts CTVECYFNCTPTC [SEQ ID NO.2] and CPDRKKKVVMVC [SEQ ID NO.3]) sharing high homology with gp120. In particular, these peptides identify a portion of gp120 V1/V2 loop, where they are separated by a spacer of three amino acids only. Despite the high global variability of this loop, the portion mapped by the Phage Display-selected peptides appears to be conserved among different viral strains, particularly the (C-S/T/E-F-basic aa-apolar aa-S/T) motif (from SEQ ID NO.2) and the (R/K-D/N-basic stretch) (from SEQ ID NO.3), suggesting a functional role for this region. The peptide sequences of the described region of gp120 (from most of the known HIV-1 strains) have been deduced from Kuiken et al. (HIV Sequence Compendium 2000 (2000), Theoretical Biology and Biophysics Group, Los Alamos National Labroatory) and are shown in Tab. 1 (SEQ ID NO.4-22 are the homologues of SEQ ID NO.2; SEQ ID NO. 23-39 are the homologues of the 3 aa-spacer; SEQ ID NO. 40-120 are the homologues of SEQ ID NO.3).

A physiological role of the region mapped by the selected peptides has never been described in literature. Since we have based the rationale of the screening on the presence of surface-bound TAT, the selected regions of gp120 can be involved in binding TAT. A TAT-gp 120 interaction has never been described before, so it is reasonable that new domains of the gp120 protein have been selected by the screening.

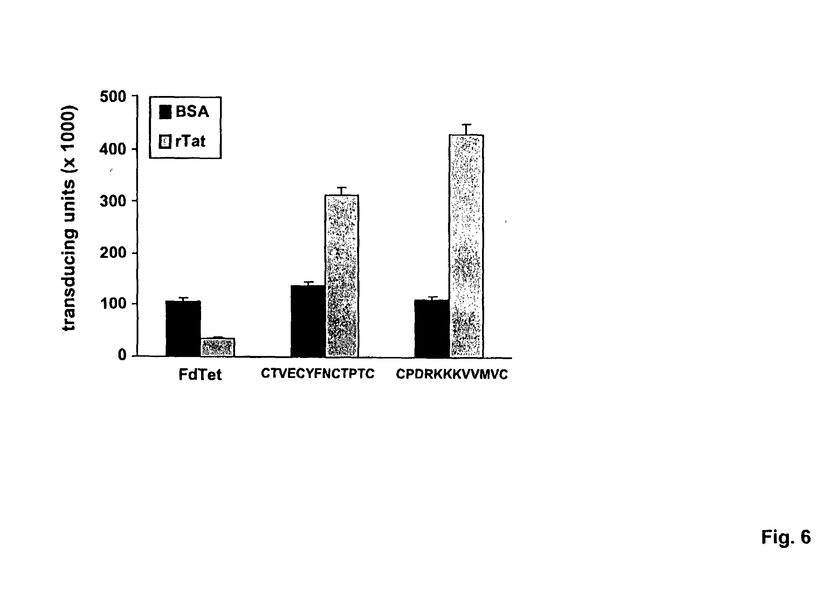

Fig. 6 shows that the phage carrying the peptides SEQ ID NO.2 and SEQ ID NO.3 specifically bind recombinant TAT protein. For this assay, we have tested single phage clones for binding rTAT-coated microwells, using an unrelated protein, the Bovine Serum Albumin (BSA), as a negative control. The result is shown in the graph, where 'Transducing Units' is a parameter indicating the number of bound phage. Binding of the phage to an insertless phage, FdTet, is shown as a negative control.

### 2.2 Properties of the peptides

In the following Example Section the peptide properties and the ability of inhibiting HIV-1 infection are shown.

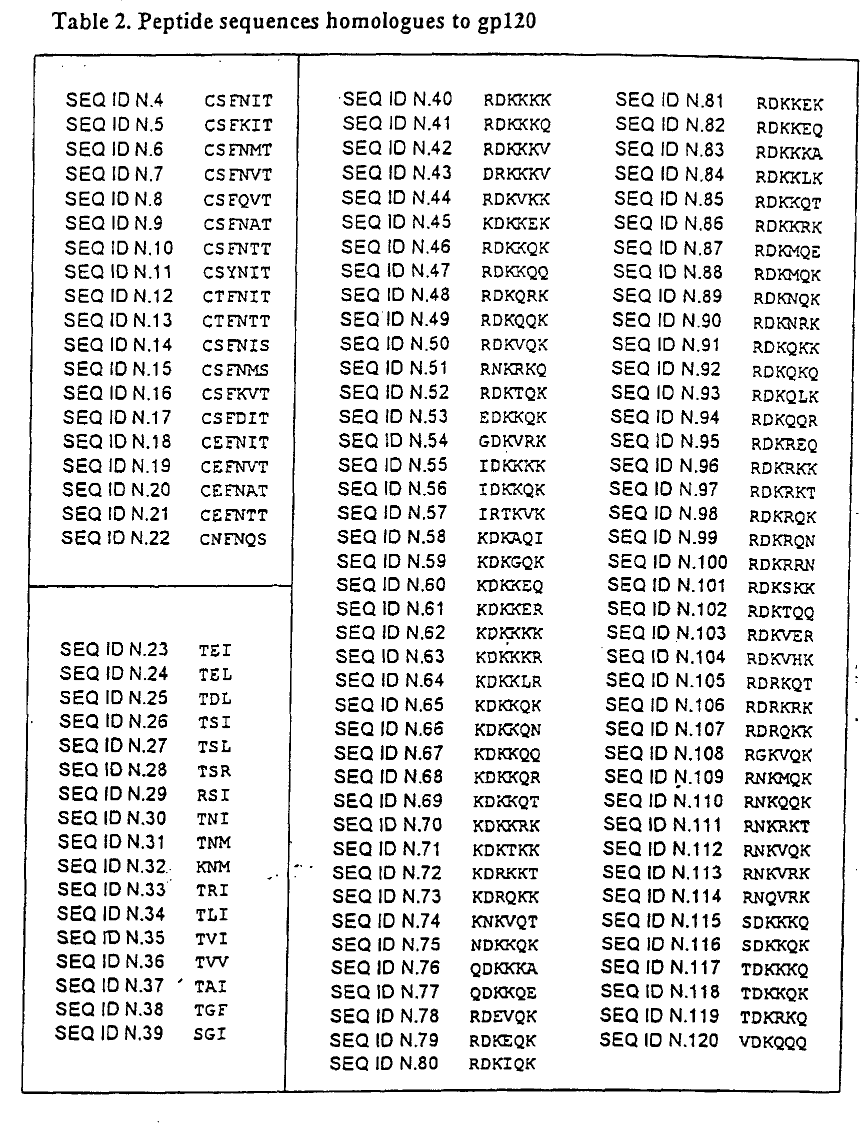

### EXAMPLES

### Example I

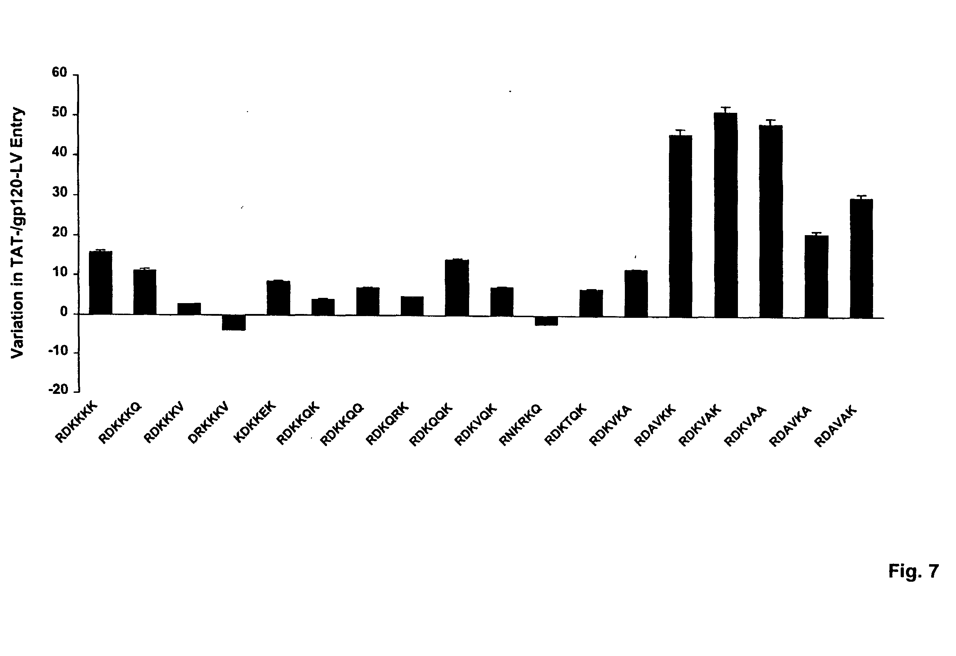

This example is illustrated in Fig. 7. Example I shows the effect of soluble peptides [the gp120 variants SEQ ID NO. 40 (RDKKKK), 41 (RDKKKQ), 42 (RDKKKV), 43 (DRKKKV), 45 (KDKKEK), 46 (RDKKQK), 47 (RDKKQQ), 48 (RDKQRK), 49 (RDKQQK), 50 (RDKVQK), 51 (RNKRKQ), 52 (RDKTQK) and the mutants SEQ ID NO. 121 (RDKVKA), 122 (RDAVKK), 123 (RDKVAK), 124 (RDKVAA), 125 (RDAVKA), 126 (RDAVAK)] on the entry of TAT-/gp120-LV into C8166 cells. TAT-/gp120-LV is a TAT-defective vector (see Methods and References therein). The number of transduced cells is evaluated as percent of GFP-positive cells after 72 h. The graph shows the percent variation of entry (i.e. of the percent variation in transduced cells) following incubation with 100 µM of the peptides. The effect of the mutant peptides is investigated to confirm the specificity of the mechanism. This example shows that in the absence of TAT none of the peptides significantly inhibits TAT-/gp120-LV entry.

### Example II

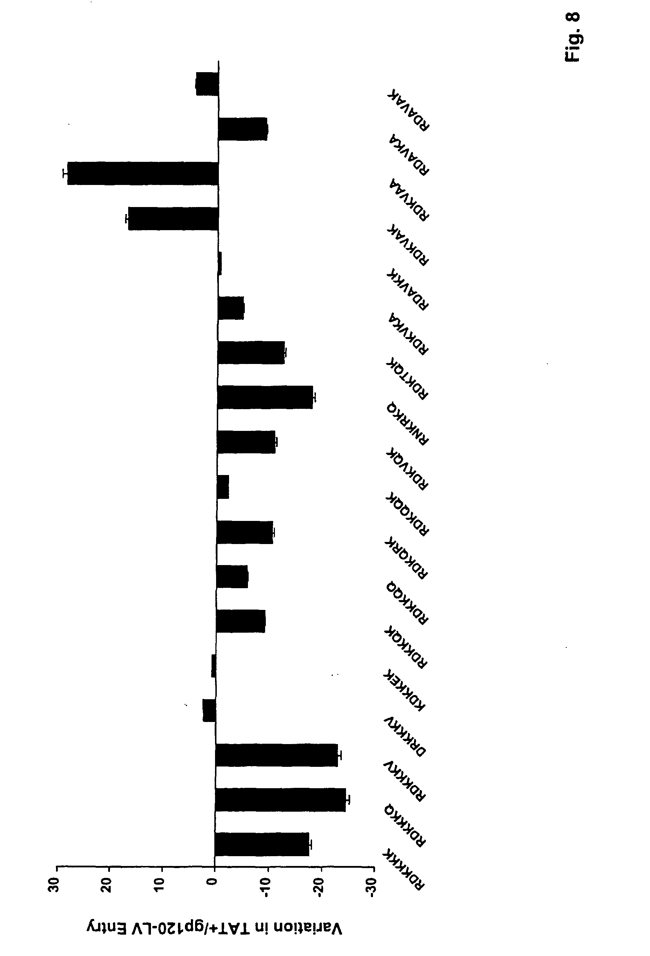

This example is illustrated in Fig. 8. Example II shows the effect of the soluble peptides described in Example I, where the entry experiment is performed with TAT+/gp120-LV. This lentiviral construct reproduces the complete HIV-1 particle, so that the experiment described in this example is closer to a true infection than Example I. As for the supernatants of HIV-1-infected cells, in TAT + /gp 120-LV preparations TAT is present, and accordingly to our model is responsible of part of the vector entry. The graph parameters are the same as in Example I. Example II shows that the entry of TAT+/gp120-LV is inhibited by some of the tested peptides, as a consequence of blocking TAT-driven entry. In particular, peptides with the SEQ ID NO. 40 (RDKKKK), 41 (RDKKKQ), 42 (RDKKKV), 51 (RNKRKQ), 52 (RDKTQK) revert the basal infection of more than 15%. To confirm the validity of the proposed model, mutant peptides do not inhibit the entry of TAT + /gp 120-LV.

### Example III

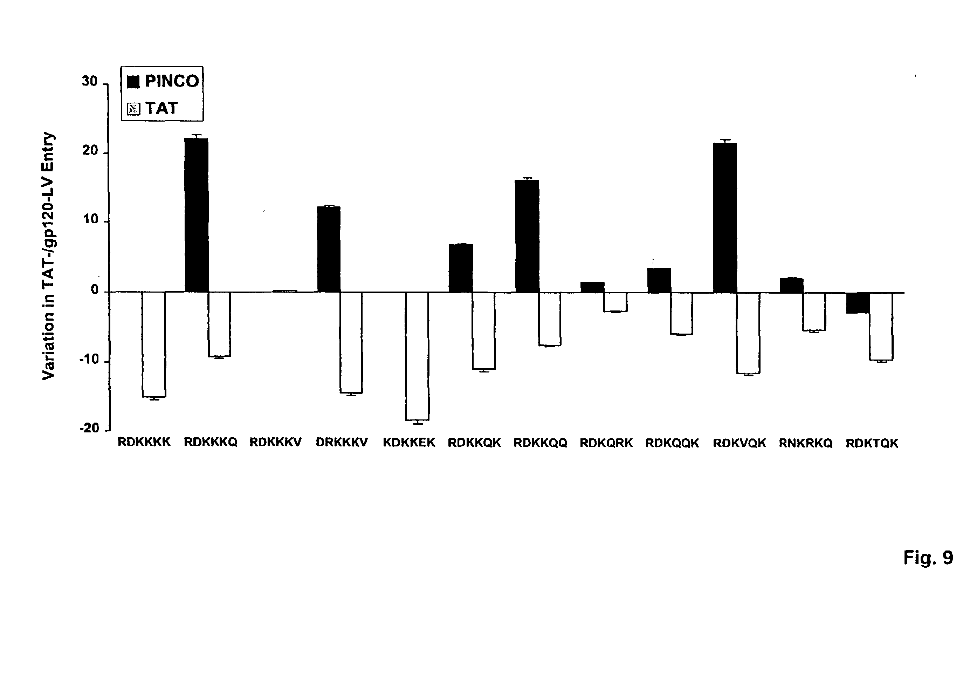

This example is illustrated in Fig. 9. Example III shows the effect of the soluble peptides described in Example I, where the entry experiment is performed with TAT-/gp120-LV, following 4 h co-culture of C8166 cells with U937/PINCO or U937/TAT cells. Said vector, as described in Example I, does not allow the expression of TAT, so that TAT is present, as detailed described before, only following co-culture with U937/TAT cells. The amount of TAT released by U937/TAT cells is 3 nM as described in Fig. 2. The graph parameters are the same as in Example I. Example III shows that none of the peptides inhibits TAT-/gp120-LV entry in the absence of TAT. On the contrary, after the cells are co-cultured with U937/TAT cells, some of the peptides inhibit vector entry. In particular, in this example the peptides with SEQ ID NO. 40 (RDKKKK), 43 (DRKKKV), 45 (KDKKEK) revert the entry of more than 15%.

### Example IV

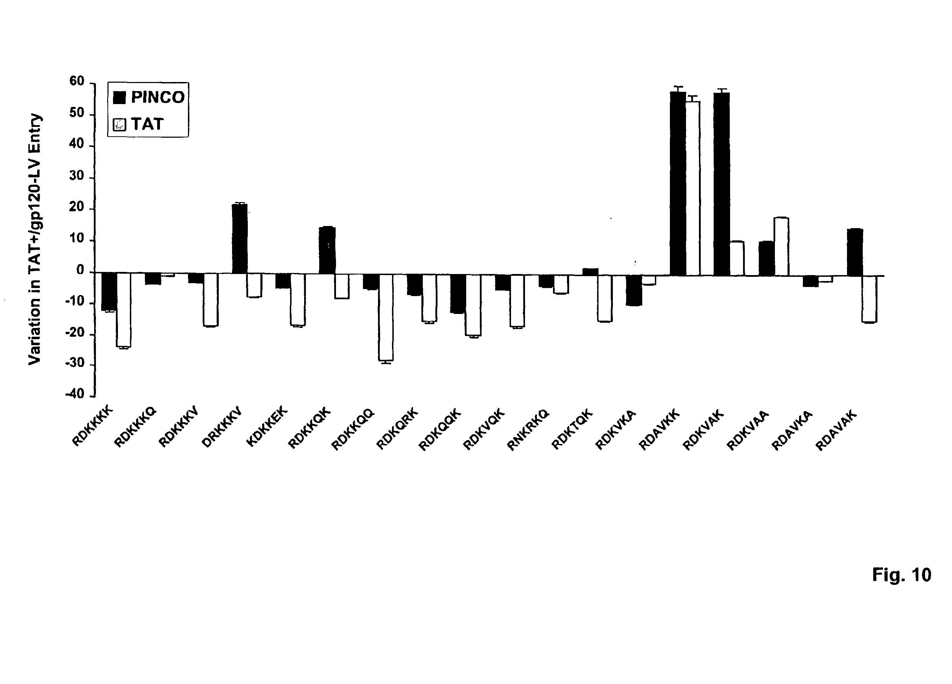

This example is illustrated in Fig. 10. Example IV shows the effect of the soluble peptides described in Example I, where the entry experiment is performed with TAT+/gp120-LV, following 4 h co-culture of C8166 cells with U937/PINCO or U937/TAT cells. Said vector, as described in Example II, allows the expression of TAT, so that TAT is already present, as detailed described before, in the vector preparation and its amount is enhanced after the co-culture with U937/TAT cells. The graph parameters are the same as in Example I. Example IV illustrates that some of the peptides slightly inhibit already the basal entry of TAT+/gp120-LV, and that this effect is potentiated by the addition of TAT due to the co-culture. In particular, peptides with SEQ ID NO. 40 (RDKKKK), 42 (RDKKKV), 45 (KDKKEK), 47 (RDKKQK), 49 (RDKQQK), 50 (RDKVQK) revert the entry of more than 15%.

### Example V

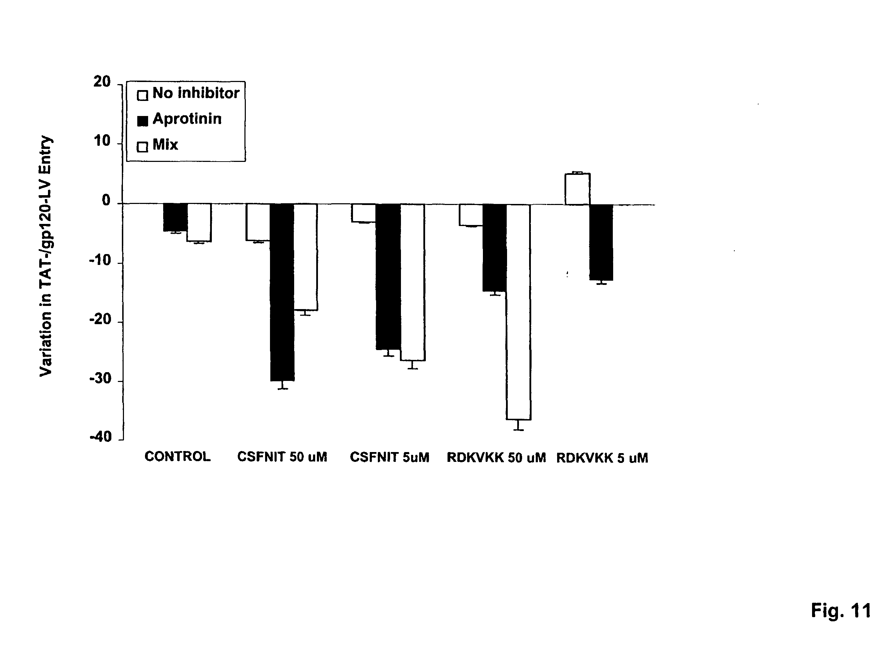

This example is illustrated in Fig. 11. Example V shows the effect of the soluble peptides SEQ ID NO. 4 (CSFINT) and 44 (RDKVKK), where the entry experiment is performed with TAT+/gp120-LV, following 4 h co-culture of C8166 cells with U937/TAT cells. These two peptides are the ones derived from the Phage Display screening, and is here evaluated their ability of inhibit vector entry at concentrations lower than 100 µM (the concentration used in all the examples previously described). To verify if the integrity of the peptides is crucial for their activity, we have incubated the cells with said peptides in the presence of different protease inhibitors (aprotinin or a mix made by aprotinin, leupeptin, bestatin, pepstatin-A, AEBSF *[4-(2-Aminoethyl) Benzenesulfonyl Fluoride Hydrochloride]* and E-64 *[(N-(N-(L-3-Transcarboxirane-2-Carbonyl)-L-Leucyl)-Agmatine)]*). Thegraph parameters are the same as in Example I. Example V shows that the efficiency of the said peptides can be increased by preventing their degradation. In particular, the graph shows that the peptides are inefficient in blocking virus entry at a concentration lower than 100 µM, but if the same peptides are incubated with said protease inhibitors their efficiency raises, being the inhibitory effect still visible at a concentration as low as 5 µM. This example illustrates a way by which the power of said peptides can be improved.

### Example VI

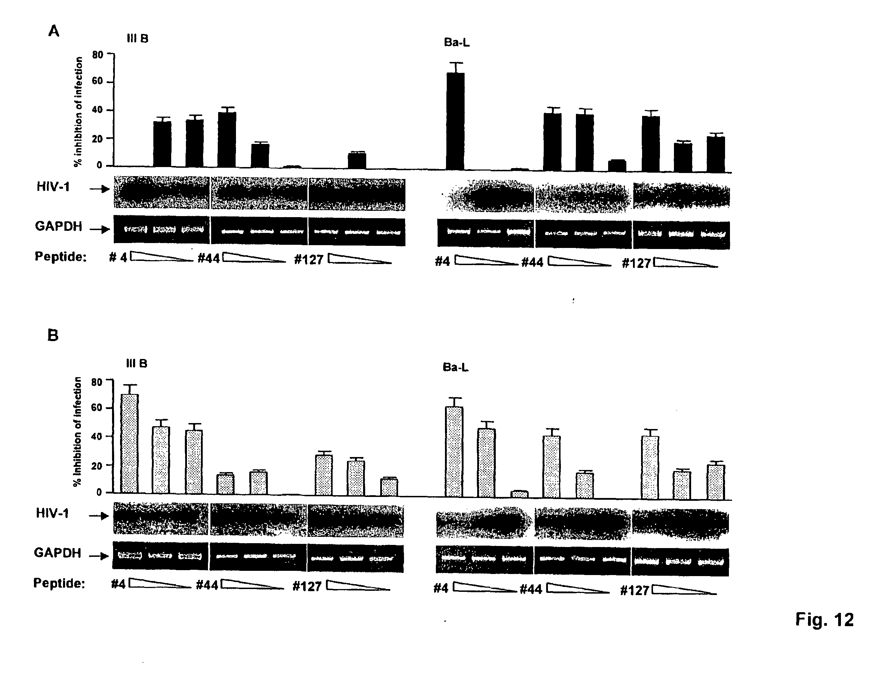

This example is illustrated in Fig. 12. Example VI shows the effect of the soluble peptides SEQ ID NO. 4 (CSFINT), 44 (RDKVKK) and the complete gp120 region SEQ ID NO.127 (CSFNITTEIRDKVKK), where the entry experiment is performed with two strains of HIV-1 on peripheral blood cells (PBMCs) derived from healthy donors. In particular, strain III B uses CXCR4 as a co-receptor (X4) and Ba-L uses CCR5 (R5). This example aims to illustrate the effect of the peptides in a true physio-pathological contest and also to demonstrate that said effect is independent from viral tropism.

In the graphs the percent inhibition of infection due to the peptides is shown, as compared to infection in the presence of 100 µM of a control peptide. PHA-stimulated PBMCs have been incubated with the peptides and immediately infected for 2 h at 37 °C. This incubation time is sufficient to evaluate virus entry, by detecting the presence of early viral transcripts into the cytoplasm of infected cells. The peptide concentrations are as follows: SEQ ID NO.4 and 44: 100-50-10 µM; SEQ ID NO. 127: 1 µM-100-10 nM. In Fig. 12A is represented the effect of the peptides on the basal infection of both viral strains, in Fig. 12B the effect of the peptides on the infection following incubation with rTAT 7 nM. A peptide-dependent inhibition of the basal infection by both HIV-1 strains is present already in the basal infection, reflecting the presence of TAT in the viral supernatants. In particular, these peptides are more efficient in reducing the basal infection by HIV-1 Ba-L than III B, reaching an inhibition from about 40% (SEQ ID NO. 44 and 127) to 70% (SEQ ID NO. 4). After cell incubation with 7 nM rTAT, the peptides have an enhanced efficacy in inhibiting the infection, and all of them show a dose-response effect, with similar outcomes on infections by both viral strains, confirming that their activity is related to the amount of membrane-bound TAT. This example shows that TAT-mediated infection is inhibited by soluble peptides blocking gp 120/TAT interaction, independently from the virus strain.

### METHODS

Methods that are not explicitly described in this disclosure and in other sections are amply reported in the scientific literature and are well within the scope of those skilled in the art.

**Cell cultures.** U937 and C8166 cells were cultured in RPMI-1640 medium supplemented with 10% heat inactivated FCS; Phoenix cells were cultured in DMEM supplemented with 10% FCS; 293T cells were cultured in Iscove's medium supplemented with 10% FCS. PBMCs from healthy donors were separated on a Ficoll gradient, and cultured in RPMI-1640 supplemented with 20% FCS and 200 U/ml Interleukin-2. Before infection, PBMCs were stimulated with 5 µg/ml PHA for 48 h.

**Preparation of U937/TAT cells.** The coding region of TAT₈₆ III B was amplified by Polymerase Chain Reaction (PCR) and subcloned into the retroviral vector PINCO (Grignani et al. (1998) supra) in the BamHI/EcoRI cloning site (Fig. 1), under the control of the LTR promoter (PINCO/TAT). Phoenix cells were transfected with either the PINCO or PINCO/TAT constructs by traditional calcium phosphate method, and viral supernatants were used to infect U937 cells.

**CAT assay**. COS-7 cells were transiently transfected with a vector carrying the gene for the enzyme chloramphenicol acetyl transferase (CAT) under a LTR promoter responsive to TAT. The cells were incubated with conditioned media from U937/TAT cells for 48 h. The cell lysates were incubated with the substrate of CAT enzyme, ¹⁴C-labeled chloramphenicol, in the presence of Acetyl CoA. The products of the reaction, i.e. the acetylated forms of ¹⁴C-chloramphenicol, were resolved by thin layer chromatography and visualized by autoradiography.

**Peptide libraries.** The CX₃CX₃CX₃C and CX₁₀C (C, cysteine; X, any amino acid) libraries were prepared using synthetic oligonucleotides, containing respectively the core sequences TGT(NNK)₃TGT(NNK)₃TGT(NNK)₃TGT and TGT(NNK)₁₀TGT (N = equal molar mixture of A, C, G, T; K = G or T). The oligonucleotides were made double stranded by PCR amplification and ligated to the N-terminus of the pIII gene of fUSE5 vector (Scott and Smith (1990, supra).

**Cell panning.** Cells were washed once in binding medium (DMEM supplemented with 20 mM HEPES and 2% FCS), and resuspended in the same medium at a concentration of 10⁶ cells/ml. 10¹⁰ TU were added to 500 µl of the cell suspension, and the mixture was incubated overnight (first round) or for 2 h (successive rounds) at 4°C with gentle rotation. Cells were washed five times in binding medium at room temperature and resuspended in 100 µl of the same medium. Phage were rescued by adding 1 ml of exponentially growing K91Kan *Escherichia coli* bacteria and incubating the mixture for 1 h at room temperature. Bacteria were diluted in 10 ml of LB medium supplemented with 0.2 µg/ml tetracycline and incubated for another 20 min at room temperature. Serial dilutions were plated on LB/agar plates containing 40 µg/ml tetracycline, and plates were incubated at 37°C overnight before colonies were counted. When a significant increase in binding was observed, peptide sequences were inferred from the sequences of the nucleotide inserts derived from PCR analysis as described (Scott and Smith (1990), supra).

**Co-cultures and incubations with rTAT.** C8166 cells were co-cultured with U937/PINCO or U937/TAT cells in 6-well transwells for 4 h at 37°C, or incubated in culture medium containing rTAT (provided by The Centralized facility for AIDS Reagents, National Institute for Biological Standards and Controls, and the UK Medical Research Council, donor Immunodiagnostic) for 15 min at 37°C. After treatment, cells were extensively washed in PBS, and subjected to immunostaining or transduction with lentiviral vectors. PBMCs were incubated with 7 nM recombinant TAT₈₆ III B for 15 min at 37°C, extensively washed and subjected to infection with HIV-1.

**Immunofluorescence.** Cells were pre-fixed in 0,1 % paraformaldeyde in PBS for 15 min on ice, stained with the NT3 2 D1 anti-TAT mAb (provided by The Centralized facility for AIDS Reagents, National Institute for Biological Standards and Controls, and the UK Medical Research Council, donor Dr. J. Karn) diluted 1:20, and revealed with a TRITC-conjugated secondary antibody following a standard protocol. Cells were then fixed in 3% paraformaldeyde in PBS and photographed with an inverted microscope.

**GST-fused TAT proteins**
The following TATHXB2 variants were produced as GST-fusion proteins as described (Mitola, S. et al. Identification of specific molecular structures of human immunodeficiency virus type 1 TAT relevant for its biological effects on vascular endothelial cells. J Virol **74**, 344-53 (2000): TAT₈₆ (full-length), TAT₇₂ (product of TAT first exon), TAT_{BasMut} (with the mutations R-49,52,53,55,56,57-A), TAT_{CysMut} (with the mutations C-22,25,27-A.

**Surface plasmon resonance (SPR) analysis.** Evaluation of interaction between TAT and gp120 III B recombinant proteins was performed using a SPR-based BlAcoreX biosensor (BlAcore AB). rTAT was diluted in 5mM maleate pH 6.0 and amine-conjugated to the dextran matrix on a CM5 sensor chip surface (BlAcore AB), in accordance with the manufacturer's protocol. Binding of recombinant gp120 III B was determined over a range of concentrations (1.25-50 nM) in 10 mM HEPES pH 7.4, 150 mM NaCl, 3 mM EDTA, 0.005% polysorbate 20 (HBS-EP buffer, BlAcore AB) with a flow rate of 5 µl/min at 25 °C. Injection volume was 20 ml and flow rate was 5 µl/min. The surface was regenerated with 0.5% SDS. Kinetic analysis was performed using the BlAevaluation 3.0.2 software.

**Transduction with HIV-1-derived lentivirus.** Vector stocks were prepared by transient co-transfection of 293T cells with the following combinations of plasmids: pRRL.hPGK.GFP.SIN-18 + pCMV.DR8.2 + pMD.G (Naldini et al. (1996), supra) (VSV-G-LV) or pEnv_{HXB} (Huang et al., J. Virol. 72 (1998), 8952-8960) (TAT+/gp120-LV); pRRL.hPGK.GFP.SIN-18 + pCMV.DR8.74 + pRSV-Rev ((Dull et al. (1998), supra) + pEnv_{HXB} (TAT⁻/gp120-LV). Vector concentration was determined by HIV-1 p24 Core profile ELISA following the manufacturer's instructions. The entry experiments were conducted on 10⁵ cells per well of a 48-well plate in the presence of 8 µg/ml polybrene. The number of GFP-positive cells was analysed by FACS.

**Infection with wild type HIV-1.** PHA-stimulated PBMCs (10⁵ cells/well of a 48-well plate) were infected with HIV-1 III B or Ba-L for 2 h at 37°C. Samples were then processed as described, with slight modifications (Schmidtmayerova et al., J. Virol. 72 (1998), 4633-4642). Briefly, cells were lysed in 200 µl of 50 mM KCI, 10 mM Tris-HCl pH 8.3, 2.5 mM MgCl₂, 0.1 mg/ml gelatin, 0.45% Nonidet P-40, 0.45% Tween 20, and 100 µg/ml proteinase K. After protein digestion (2 h at 56°C) and inactivation of the proteinase (10 min at 95°C), 0.1 µl of cell lysate was subjected to 35 cycles of PCR, with an annealing temperature of 58°C. Amplified DNA was analyzed by Southern blot hybridization with a ³²P-labeled probe, and quantified by densitometry. The following primers and probe were used:

SEQUENCES MENTIONED IN THIS INVENTION

SEQ ID No.1 TAT III B
1 - MIPVDPRLEP WKHPGSQPKT ACTNCYCKKC CFHCQVCFIT
KALGISYGRK KRRQRRRAHQ NSQTHQASLS KQPTSQPRGD PTGPKE -
86

SEQ ID No. 2 Phage insert 1 - CTVECYFNCTPTC - 13
SEQ ID No. 3 Phage insert 1 - CPDRKKKVVMVC - 12
SEQ ID No. 4-120 Peptide sequences similar to gp120 (see
Table 2); in particular, soluble peptides cited in the
Examples:
SEQ ID No. 4 1 - CSFNIT - 6
SEQ ID No. 40 1 - RDKKKK - 6
SEQ ID No. 41 1 - RDKKKQ - 6
SEQ ID No. 42 1 - RDKKKV - 6
SEQ ID No. 43 1 - DRKKKV - 6
SEQ ID No. 44 1 - RDKVKK - 6
SEQ ID No. 45 1 - KDKKEK - 6
SEQ ID No. 46 1 - RDKKQK - 6
SEQ ID No. 47 1 - RDKKQQ - 6
SEQ ID No. 48 1 - RDKQRK - 6
SEQ ID No. 49 1 - RDKQQK - 6
SEQ ID No. 50 1 - RDKVQK - 6
SEQ ID No. 51 1 - RNKRKQ - 6
SEQ ID No. 52 1 - RDKTQK - 6
SEQ ID No. 121-126 Synthetic peptides corresponding to SEQ
ID No. 44 with single or double mutations in basic
residues (K → A), in particular:
SEQ ID No. 121 1 - RDKVKA - 6
SEQ ID No. 122 1 - RDAVKK - 6
SEQ ID No. 123 1 - RDKVAK - 6
SEQ ID No. 124 1 - RDKVAA - 6
SEQ ID No. 125 1 - RDAVKA - 6
SEQ ID No. 126 1 - RDAVAK - 6
SEQ ID No. 127 Synthetic peptide corresponding to the selected
region of gp120
1 - CSFNIT-TEI-RDKVKK - 15

R/U5 sense primer 5'-GGCTAACTAGGGAACCCACTG-3' (SEQ ID NO.
128),

antisense primer 5'-CTGCTAGAGATTTTCCACACTGAC-3' (SEQ ID NO.
129), and

probe 5'-TGTGTGCCCGTCTGTTGTGTG-3' (SEQ ID NO. 130);
GAPDH, sense primer 5'-ACCACAGTCCATGCCATCAC-3' (SEQ ID NO.
131), and

antisense primer 5'-TCCACCACCCTGTTGCTGTAG-3' (SEQ ID NO. 132).

Having now fully described the invention, it will be appreciated by those skilled in the art that the same can be performed within a wide range of equivalent parameters, concentrations, and conditions without departing from the spirit and scope of the invention and without due experimentation.

While this invention has been described in connection with specific embodiments thereof, it will be understood that it is capable of further modifications. This application is intended to cover any variations, uses, or adaptations of the inventions following, in general, the principles of the invention and including such departures from the present disclosure as come within known or customary practice within the art to which the invention pertains and as may be applied to the essential features hereinbefore set forth as follows in the scope of the appended claims. All references cited herein, including journal articles or abstracts, published or corresponding U.S. or foreign patent applications, issued U.S. o foreign patents, or any other references, are entirely incorporated by reference therein, including all data, tables, figures, and text presented in the cited references. Additionally, the entire contents of the references cited within the references cited herein are also entirely incorporated by reference.

Reference to known method steps, conventional method steps, known methods or conventional methods is not in any way an admission that any aspect, description or embodiment of the present invention is disclosed, taught or suggested in the relevant art. The foregoing description of the specific embodiments will so fully reveal the general nature of the invention that others can, by applying knowledge within the skill of the art (including the contents of the references cited herein), readily modify and/or adapt for various applications such specific embodiments, without undue experimentation, without departing from the general concept of the present invention. Therefore, such adaptations and modifications are intended to be within the meaning and range of equivalents of the disclosed embodiments, based on the teaching and guidance presented therein. It is to be understood that the phraseology or terminology herein is for the purpose of description and not of limitation, such that the terminology or phraseology of the present specification is to be interpreted by the skilled artisan in light of the teaching and guidance presented herein, in combination with the knowledge of one of ordinary skill in the art.

## Claims

1. Use of inhibitors of the interaction between HIV-1 TAT protein and HIV-1 gp120 for inhibiting the entry of HIV-1 into a host cell.

2. Use of claim 1, wherein the inhibitor binds to TAT.

3. Use of claim 2, wherein the inhibitor is a peptide.

4. Use of claim 3, wherein the peptide is homologous to the gp 120 V1/V2 region.

5. Use of claim 3 or 4, wherein the pepide is selected from:
(a) CTVECYFNCTPTC (SEQ ID No. 2)
(b) CPDRKKKVVMVC (SEQ ID No. 3)
(c) CSFNITTEIRDKVKK (SEQ ID No. 127)
(d) a peptide comprising at least 5 contiguous amino acids from a peptide, selected from the group consisting of peptides (a) - (c),
(e) a peptide which has a sequence identity of at least 80 % to the amino acid sequence of a peptide selected from the group consisting of peptides (a) - (d).

6. Use of claim 3 or 4, wherein the peptide is selected from:
(a) RDKKKK (SEQ ID No. 40),
(b) RDKKKQ (SEQ ID No. 41),
(c) RDKKKV (SEQ ID No. 42),
(d) RNKRKQ (SEQ ID No. 51),
(e) RDKTQK (SEQ ID No. 52),
(f) DRKKKV (SEQ ID No. 43),
(g) KDKKEK (SEQ ID No. 45),
(h) RDKQQK (SEQ ID No. 49),
(i) RDKVQK (SEQ ID No. 50),
(j) CSFNIT (SEQ ID No. 4),
(k) RDKVKK (SEQ ID No. 44),
(I) a peptide comprising at least 5 contiguous amino acids from a peptide selected from the group consisiting of peptides (a) -(k),
(m) a peptide which has an identity of at least 80 % to the amino acid sequence of peptide selected from the group consisting of peptides (a) - (l).

7. Use of claim 1, wherein the inhibitor binds to gf120.

8. Use of any one of claims 1 to 7, wherein the host cell is a human cell.

9. Use fo any one of claims 1 to 8 for the manufacture of a medicament for the treatment of HIV-1 infections.

10. Use of claim 9 for the treatment of infections by M-tropic and L-tropic HIV-1 strains.

11. A method for identifying and/or **characterizing** a compound which inhibits the entry of HIV-1 into a host cell comprising
(i) providing at least one compound to be tested and
(ii) determining if the compound is capable of inhibiting the interaction between HIV-1 TAT protein and HIV-1 gp120.

12. The method of claaim 11, wherein a plurality of compounds is tested in parallel or sequential.

13. The method of claim 12, wherein a compound library is tested.

14. The method of any one of claims 11 to 13 which is a cellular-based assay.

15. The method of any one of claims 11 to 14 which is a molecular-based assay.

16. The method of any one of claims 11 to 15, wherein a compound which has been identified as an inhibitor or a compound desired therefrom is formulated as a pharmaceutical composition.

17. A pharmaceutical composition comprising as an active ingredient at least one inhibitor of the interaction between HIV-1 TAT protein and HIV-1 gp 120 and optionally pharmaceutically acceptable carriers, diluents and/or adjuvants.

18. The pharmaceutical composition of claim 17, wherein the inhibitor is defined as in claims 2 to 7.

19. A peptide which is selected from:
(a) CTVECYFNCTPTC (SEQ ID No. 2)
(b) CPDRKKKVVMVC (SEQ ID No. 3)
(c) CSFNITTEIRDKVKK (SEQ ID No. 127)
(d) a peptide comprising at least 5 contiguous amino acids from a peptide, selected from the group consisting of peptides (a) - (c),
(e) a peptide which has a sequence identity of at least 80 % to the amino acid sequence of a peptide selected from the group consisting of peptides (a) - (d).

20. A peptide which is selected from:
(a) RDKKKK (SEQ ID No. 40),
(b) RDKKKQ (SEQ ID No. 41),
(c) RDKKKV (SEQ ID No. 42),
(d) RNKRKQ (SEQ ID No. 51),
(e) RDKTQK (SEQ ID No. 52),
(f) DRKKKV (SEQ ID No. 43),
(g) KDKKEK (SEQ ID No. 45),
(h) RDKQQK (SEQ ID No. 49),
(i) RDKVQK (SEQ ID No. 50),
(j) CSFNIT (SEQ ID No. 4),
(k) RDKVKK (SEQ ID No. 44),
(l) a peptide comprising at least 5 contiguous amino acids from a peptide selected from the group consisiting of peptides (a) -(k),
(m) a peptide which has an identity of at least 80 % to the amino acid sequence of peptide selected from the group consisting of peptides (a) - (l).

21. Peptide combination comprising at least two peptides with the sequences shown in SEO ID NO. 4-120.


SEQUENCE LISTING

<110> Università di Torino

<120> A novel mechanism for HIV-1 entry into host cells and
      peptides inhibiting this mechanism

<130> 28665PEP1_RI

<140> EP02028510.2
<141> 2002-12-19

<160> 132

<170> PatentIn Ver. 2.1

<210> 1
<211> 86
<212> PRT
<213> HIV

<400> 1
Met Ile Pro Val Asp Pro Arg Leu Glu Pro Trp Lys His Pro Gly Ser
  1               5                  10                  15

Gln Pro Lys Thr Ala Cys Thr Asn Cys Tyr Cys Lys Lys Cys Cys Phe
             20                  25                  30

His Cys Gln Val Cys Phe Ile Thr Lys Ala Leu Gly Ile Ser Tyr Gly
         35                  40                  45

Arg Lys Lys Arg Arg Gln Arg Arg Arg Ala His Gln Asn Ser Gln Thr
     50                  55                  60

His Gln Ala Ser Leu Ser Lys Gln Pro Thr Ser Gln Pro Arg Gly Asp
 65                  70                  75                  80

Pro Thr Gly Pro Lys Glu
                 85

<210> 2
<211> 13
<212> PRT
<213> HIV

<400> 2

Cys Thr Val Glu Cys Tyr Phe Asn Cys Thr Pro Thr Cys
1               5                   10

<210> 3
<211> 12
<212> PRT
<213> HIV

<400> 3
Cys Pro Asp Arg Lys Lys Lys Val Val Met Val Cys
1               5                   10

<210> 4
<211> 6
<212> PRT
<213> Artificial Sequence

<220>
<223> Description of Artificial Sequence:peptidic
      inhibitor

<400> 4
Cys Ser Phe Asn Ile Thr
1               5

<210> 5
<211> 6
<212> PRT
<213> Artificial Sequence

<220>
<223> Description of Artificial Sequence:peptidic
      inhibitor

<400> 5
Cys Ser Phe Lys Ile Thr
1               5

<210> 6
<211> 6

<212> PRT
<213> Artificial Sequence

<220>
<223> Description of Artificial Sequence:peptidic
      inhibitor

<400> 6
Cys Ser Phe Asn Met Thr
  1               5

<210> 7
<211> 6
<212> PRT
<213> Artificial Sequence

<220>
<223> Description of Artificial Sequence:peptidic
      inhibitor

<400> 7
Cys Ser Phe Asn Val Thr
  1               5

<210> 8
<211> 6
<212> PRT
<213> Artificial Sequence

<220>
<223> Description of Artificial Sequence:peptidic
      inhibitor

<400> 8
Cys Ser Phe Gln Val Thr
  1               5

<210> 9
<211> 6
<212> PRT
<213> Artificial Sequence

<220>
<223> Description of Artificial Sequence:peptidic
      inhibitor

<400> 9
Cys Ser Phe Asn Ala Thr
  1               5

<210> 10
<211> 6
<212> PRT
<213> Artificial Sequence

<220>
<223> Description of Artificial Sequence:peptidic
      inhibitor

<400> 10
Cys Ser Phe Asn Thr Thr
  1               5

<210> 11
<211> 6
<212> PRT
<213> Artificial Sequence

<220>
<223> Description of Artificial Sequence:peptidic
      inhibitor

<400> 11
Cys Ser Tyr Asn Ile Thr
  1               5

<210> 12
<211> 6
<212> PRT
<213> Artificial Sequence

<220>
<223> Description of Artificial Sequence:peptidic
      inhibitor

<400> 12
Cys Thr Phe Asn Ile Thr
1               5

<210> 13
<211> 6
<212> PRT
<213> Artificial Sequence

<220>
<223> Description of Artificial Sequence:peptidic
      inhibitor

<400> 13
Cys Thr Phe Asn Thr Thr
1               5

<210> 14
<211> 6
<212> PRT
<213> Artificial Sequence

<220>
<223> Description of Artificial Sequence:peptidic
      inhibitor

<400> 14
Cys Ser Phe Asn Ile Ser
1               5

<210> 15
<211> 6
<212> PRT
<213> Artificial Sequence

<220>
<223> Description of Artificial Sequence:peptidic
      inhibitor

<400> 15
Cys Ser Phe Asn Met Ser

1                   5

<210> 16
<211> 6
<212> PRT
<213> Artificial Sequence

<220>
<223> Description of Artificial Sequence:peptidic
      inhibitor

<400> 16
Cys Ser Phe Lys Val Thr
  1               5

<210> 17
<211> 6
<212> PRT
<213> Artificial Sequence

<220>
<223> Description of Artificial Sequence:peptidic
      inhibitor

<400> 17
Cys Ser Phe Asp Ile Thr
  1               5

<210> 18
<211> 6
<212> PRT
<213> Artificial Sequence

<220>
<223> Description of Artificial Sequence:peptidic
      inhibitor

<400> 18
Cys Glu Phe Asn Ile Thr
  1               5

<210> 19
<211> 6
<212> PRT
<213> Artificial Sequence

<220>
<223> Description of Artificial Sequence:peptidic
      inhibitor

<400> 19
Cys Glu Phe Asn Val Thr
  1               5

<210> 20
<211> 6
<212> PRT
<213> Artificial Sequence

<220>
<223> Description of Artificial Sequence:peptidic
      inhibitor

<400> 20
Cys Glu Phe Asn Ala Thr
  1               5

<210> 21
<211> 6
<212> PRT
<213> Artificial Sequence

<220>
<223> Description of Artificial Sequence:peptidic
      inhibitor

<400> 21
Cys Glu Phe Asn Thr Thr
  1               5

<210> 22
<211> 6

<212> PRT
<213> Artificial Sequence

<220>
<223> Description of Artificial Sequence:peptidic
      inhibitor

<400> 22
Cys Asn Phe Asn Gln Ser
  1               5

<210> 23
<211> 3
<212> PRT
<213> Artificial Sequence

<220>
<223> Description of Artificial Sequence:peptidic
      inhibitor

<400> 23
Thr Glu Ile
  1

<210> 24
<211> 3
<212> PRT
<213> Artificial Sequence

<220>
<223> Description of Artificial Sequence:peptidic
      inhibitor

<400> 24
Thr Glu Leu
  1

<210> 25
<211> 3
<212> PRT
<213> Artificial Sequence

<220>
<223> Description of Artificial Sequence:peptidic
      inhibitor

<400> 25
Thr Asp Leu
  1

<210> 26
<211> 3
<212> PRT
<213> Artificial Sequence

<220>
<223> Description of Artificial Sequence:peptidic
      inhibitor

<400> 26
Thr Ser Ile
  1

<210> 27
<211> 3
<212> PRT
<213> Artificial Sequence

<220>
<223> Description of Artificial Sequence:peptidic
      inhibitor

<400> 27
Thr Ser Leu
  1

<210> 28
<211> 3
<212> PRT
<213> Artificial Sequence

<220>
<223> Description of Artificial Sequence:peptidic
      inhibitor

<400> 28
Thr Ser Arg
1

<210> 29
<211> 3
<212> PRT
<213> Artificial Sequence

<220>
<223> Description of Artificial Sequence:peptidic
      inhibitor

<400> 29
Arg Ser Ile
1

<210> 30
<211> 3
<212> PRT
<213> Artificial Sequence

<220>
<223> Description of Artificial Sequence:peptidic
      inhibitor

<400> 30
Thr Asn Ile
1

<210> 31
<211> 3
<212> PRT
<213> Artificial Sequence

<220>
<223> Description of Artificial Sequence:peptidic
      inhibitor

<400> 31
Thr Asn Met

1

<210> 32
<211> 3
<212> PRT
<213> Artificial Sequence

<220>
<223> Description of Artificial Sequence:peptidic
      inhibitor

<400> 32
Lys Asn Met
  1

<210> 33
<211> 3
<212> PRT
<213> Artificial Sequence

<220>
<223> Description of Artificial Sequence:peptidic
      inhibitor

<400> 33
Thr Arg Ile
  1

<210> 34
<211> 3
<212> PRT
<213> Artificial Sequence

<220>
<223> Description of Artificial Sequence:peptidic
      inhibitor

<400> 34
Thr Leu Ile
  1

<210> 35
<211> 3
<212> PRT
<213> Artificial Sequence

<220>
<223> Description of Artificial Sequence:peptidic
      inhibitor

<400> 35
Thr Val Ile
  1

<210> 36
<211> 3
<212> PRT
<213> Artificial Sequence

<220>
<223> Description of Artificial Sequence:peptidic
      inhibitor

<400> 36
Thr Val Val
  1

<210> 37
<211> 3
<212> PRT
<213> Artificial Sequence

<220>
<223> Description of Artificial Sequence:peptidic
      inhibitor

<400> 37
Thr Ala Ile
  1

<210> 38
<211> 3

<212> PRT
<213> Artificial Sequence

<220>
<223> Description of Artificial Sequence:peptidic
      inhibitor

<400> 38
Thr Gly Phe
  1

<210> 39
<211> 3
<212> PRT
<213> Artificial Sequence

<220>
<223> Description of Artificial Sequence:peptidic
      inhibitor

<400> 39
Ser Gly Ile
  1

<210> 40
<211> 6
<212> PRT
<213> Artificial Sequence

<220>
<223> Description of Artificial Sequence:peptidic
      inhibitor

<400> 40
Arg Asp Lys Lys Lys Lys
  1               5

<210> 41
<211> 6
<212> PRT
<213> Artificial Sequence

<220>
<223> Description of Artificial Sequence:peptidic
      inhibitor

<400> 41
Arg Asp Lys Lys Lys Gln
  1               5

<210> 42
<211> 6
<212> PRT
<213> Artificial Sequence

<220>
<223> Description of Artificial Sequence:peptidic
      inhibitor

<400> 42
Arg Asp Lys Lys Lys Val
  1               5

<210> 43
<211> 6
<212> PRT
<213> Artificial Sequence

<220>
<223> Description of Artificial Sequence:peptidic
      inhibitor

<400> 43
Asp Arg Lys Lys Lys Val
  1               5

<210> 44
<211> 6
<212> PRT
<213> Artificial Sequence

<220>
<223> Description of Artificial Sequence:peptidic
      inhibitor

<400> 44
Arg Asp Lys Val Lys Lys
1               5

<210> 45
<211> 6
<212> PRT
<213> Artificial Sequence

<220>
<223> Description of Artificial Sequence:peptidic
      inhibitor

<400> 45
Lys Asp Lys Lys Glu Lys
1               5

<210> 46
<211> 6
<212> PRT
<213> Artificial Sequence

<220>
<223> Description of Artificial Sequence:peptidic
      inhibitor

<400> 46
Arg Asp Lys Lys Gln Lys
1               5

<210> 47
<211> 6
<212> PRT
<213> Artificial Sequence

<220>
<223> Description of Artificial Sequence:peptidic
      inhibitor

<400> 47
Arg Asp Lys Lys Gln Gln

1                 5

<210> 48
<211> 6
<212> PRT
<213> Artificial Sequence

<220>
<223> Description of Artificial Sequence:peptidic
      inhibitor

<400> 48
Arg Asp Lys Gln Arg Lys
  1               5

<210> 49
<211> 6
<212> PRT
<213> Artificial Sequence

<220>
<223> Description of Artificial Sequence:peptidic
      inhibitor

<400> 49
Arg Asp Lys Gln Gln Lys
  1               5

<210> 50
<211> 6
<212> PRT
<213> Artificial Sequence

<220>
<223> Description of Artificial Sequence:peptidic
      inhibitor

<400> 50
Arg Asp Lys Val Gln Lys
  1               5

<210> 51
<211> 6
<212> PRT
<213> Artificial Sequence

<220>
<223> Description of Artificial Sequence:peptidic
      inhibitor

<400> 51
Arg Asn Lys Arg Lys Gln
  1               5

<210> 52
<211> 6
<212> PRT
<213> Artificial Sequence

<220>
<223> Description of Artificial Sequence:peptidic
      inhibitor

<400> 52
Arg Asp Lys Thr Gln Lys
  1               5

<210> 53
<211> 6
<212> PRT
<213> Artificial Sequence

<220>
<223> Description of Artificial Sequence:peptidic
      inhibitor

<400> 53
Glu Asp Lys Lys Gln Lys
  1               5

<210> 54
<211> 6

<212> PRT
<213> Artificial Sequence

<220>
<223> Description of Artificial Sequence:peptidic
      inhibitor

<400> 54
Gly Asp Lys Val Arg Lys
  1               5

<210> 55
<211> 6
<212> PRT
<213> Artificial Sequence

<220>
<223> Description of Artificial Sequence:peptidic
      inhibitor

<400> 55
Ile Asp Lys Lys Lys Lys
  1               5

<210> 56
<211> 6
<212> PRT
<213> Artificial Sequence

<220>
<223> Description of Artificial Sequence:peptidic
      inhibitor

<400> 56
Ile Asp Lys Lys Gln Lys
  1               5

<210> 57
<211> 6
<212> PRT
<213> Artificial Sequence

<220>
<223> Description of Artificial Sequence:peptidic
      inhibitor

<400> 57
Ile Arg Thr Lys Val Lys
  1               5

<210> 58
<211> 6
<212> PRT
<213> Artificial Sequence

<220>
<223> Description of Artificial Sequence:peptidic
      inhibitor

<400> 58
Lys Asp Lys Ala Gln Ile
  1               5

<210> 59
<211> 6
<212> PRT
<213> Artificial Sequence

<220>
<223> Description of Artificial Sequence:peptidic
      inhibitor

<400> 59
Lys Asp Lys Gly Gln Lys
  1               5

<210> 60
<211> 6
<212> PRT
<213> Artificial Sequence

<220>
<223> Description of Artificial Sequence:peptidic
      inhibitor

<400> 60
Lys Asp Lys Lys Glu Gln
1               5

<210> 61
<211> 6
<212> PRT
<213> Artificial Sequence

<220>
<223> Description of Artificial Sequence:peptidic
      inhibitor

<400> 61
Lys Asp Lys Lys Glu Arg
1               5

<210> 62
<211> 6
<212> PRT
<213> Artificial Sequence

<220>
<223> Description of Artificial Sequence:peptidic
      inhibitor

<400> 62
Lys Asp Lys Lys Lys Lys
1               5

<210> 63
<211> 6
<212> PRT
<213> Artificial Sequence

<220>
<223> Description of Artificial Sequence:peptidic
      inhibitor

<400> 63
Lys Asp Lys Lys Lys Arg

1                   5



<210> 64
<211> 6
<212> PRT
<213> Artificial Sequence

<220>
<223> Description of Artificial Sequence:peptidic
      inhibitor

<400> 64
Lys Asp Lys Lys Leu Arg
  1               5



<210> 65
<211> 6
<212> PRT
<213> Artificial Sequence

<220>
<223> Description of Artificial Sequence:peptidic
      inhibitor

<400> 65
Lys Asp Lys Lys Gln Lys
  1               5



<210> 66
<211> 6
<212> PRT
<213> Artificial Sequence

<220>
<223> Description of Artificial Sequence:peptidic
      inhibitor

<400> 66
Lys Asp Lys Lys Gln Asn
  1               5

<210> 67
<211> 6
<212> PRT
<213> Artificial Sequence

<220>
<223> Description of Artificial Sequence:peptidic
      inhibitor

<400> 67
Lys Asp Lys Lys Gln Gln
  1               5

<210> 68
<211> 6
<212> PRT
<213> Artificial Sequence

<220>
<223> Description of Artificial Sequence:peptidic
      inhibitor

<400> 68
Lys Asp Lys Lys Gln Arg
  1               5

<210> 69
<211> 6
<212> PRT
<213> Artificial Sequence

<220>
<223> Description of Artificial Sequence:peptidic
      inhibitor

<400> 69
Lys Asp Lys Lys Gln Thr
  1               5

<210> 70
<211> 6

<212> PRT
<213> Artificial Sequence

<220>
<223> Description of Artificial Sequence:peptidic
      inhibitor

<400> 70
Lys Asp Lys Lys Arg Lys
  1               5

<210> 71
<211> 6
<212> PRT
<213> Artificial Sequence

<220>
<223> Description of Artificial Sequence:peptidic
      inhibitor

<400> 71
Lys Asp Lys Thr Lys Lys
  1               5

<210> 72
<211> 6
<212> PRT
<213> Artificial Sequence

<220>
<223> Description of Artificial Sequence:peptidic
      inhibitor

<400> 72
Lys Asp Arg Lys Lys Thr
  1               5

<210> 73
<211> 6
<212> PRT
<213> Artificial Sequence

<220>
<223> Description of Artificial Sequence:peptidic
      inhibitor

<400> 73
Lys Asp Arg Gln Lys Lys
  1               5

<210> 74
<211> 6
<212> PRT
<213> Artificial Sequence

<220>
<223> Description of Artificial Sequence:peptidic
      inhibitor

<400> 74
Lys Asn Lys Val Gln Thr
  1               5

<210> 75
<211> 6
<212> PRT
<213> Artificial Sequence

<220>
<223> Description of Artificial Sequence:peptidic
      inhibitor

<400> 75
Asn Asp Lys Lys Gln Lys
  1               5

<210> 76
<211> 6
<212> PRT
<213> Artificial Sequence

<220>
<223> Description of Artificial Sequence:peptidic
      inhibitor

<400> 76
Gln Asp Lys Lys Lys Ala
1               5

<210> 77
<211> 6
<212> PRT
<213> Artificial Sequence

<220>
<223> Description of Artificial Sequence:peptidic
      inhibitor

<400> 77
Gln Asp Lys Lys Gln Glu
1               5

<210> 78
<211> 6
<212> PRT
<213> Artificial Sequence

<220>
<223> Description of Artificial Sequence:peptidic
      inhibitor

<400> 78
Arg Asp Glu Val Gln Lys
1               5

<210> 79
<211> 6
<212> PRT
<213> Artificial Sequence

<220>
<223> Description of Artificial Sequence:peptidic
      inhibitor

<400> 79
Arg Asp Lys Glu Gln Lys

1                5

<210> 80
<211> 6
<212> PRT
<213> Artificial Sequence

<220>
<223> Description of Artificial Sequence:peptidic
      inhibitor

<400> 80
Arg Asp Lys Ile Gln Lys
  1               5

<210> 81
<211> 6
<212> PRT
<213> Artificial Sequence

<220>
<223> Description of Artificial Sequence:peptidic
      inhibitor

<400> 81
Arg Asp Lys Lys Glu Lys
  1               5

<210> 82
<211> 6
<212> PRT
<213> Artificial Sequence

<220>
<223> Description of Artificial Sequence:peptidic
      inhibitor

<400> 82
Arg Asp Lys Lys Glu Gln
  1               5

<210> 83
<211> 6
<212> PRT
<213> Artificial Sequence

<220>
<223> Description of Artificial Sequence:peptidic
      inhibitor

<400> 83
Arg Asp Lys Lys Lys Ala
  1               5

<210> 84
<211> 6
<212> PRT
<213> Artificial Sequence

<220>
<223> Description of Artificial Sequence:peptidic
      inhibitor

<400> 84
Arg Asp Lys Lys Leu Lys
  1               5

<210> 85
<211> 6
<212> PRT
<213> Artificial Sequence

<220>
<223> Description of Artificial Sequence:peptidic
      inhibitor

<400> 85
Arg Asp Lys Lys Gln Thr
  1               5

<210> 86
<211> 6

<212> PRT
<213> Artificial Sequence

<220>
<223> Description of Artificial Sequence:peptidic
      inhibitor

<400> 86
Arg Asp Lys Lys Arg Lys
  1               5

<210> 87
<211> 6
<212> PRT
<213> Artificial Sequence

<220>
<223> Description of Artificial Sequence:peptidic
      inhibitor

<400> 87
Arg Asp Lys Met Gln Glu
  1               5

<210> 88
<211> 6
<212> PRT
<213> Artificial Sequence

<220>
<223> Description of Artificial Sequence:peptidic
      inhibitor

<400> 88
Arg Asp Lys Met Gln Lys
  1               5

<210> 89
<211> 6
<212> PRT
<213> Artificial Sequence

<220>
<223> Description of Artificial Sequence:peptidic
      inhibitor

<400> 89
Arg Asp Lys Asn Gln Lys
  1               5

<210> 90
<211> 6
<212> PRT
<213> Artificial Sequence

<220>
<223> Description of Artificial Sequence:peptidic
      inhibitor

<400> 90
Arg Asp Lys Asn Arg Lys
  1               5

<210> 91
<211> 6
<212> PRT
<213> Artificial Sequence

<220>
<223> Description of Artificial Sequence:peptidic
      inhibitor

<400> 91
Arg Asp Lys Gln Lys Lys
  1               5

<210> 92
<211> 6
<212> PRT
<213> Artificial Sequence

<220>
<223> Description of Artificial Sequence:peptidic
      inhibitor

<400> 92
Arg Asp Lys Gln Lys Gln
1               5

<210> 93
<211> 6
<212> PRT
<213> Artificial Sequence

<220>
<223> Description of Artificial Sequence:peptidic
      inhibitor

<400> 93
Arg Asp Lys Gln Leu Lys
1               5

<210> 94
<211> 6
<212> PRT
<213> Artificial Sequence

<220>
<223> Description of Artificial Sequence:peptidic
      inhibitor

<400> 94
Arg Asp Lys Gln Gln Arg
1               5

<210> 95
<211> 6
<212> PRT
<213> Artificial Sequence

<220>
<223> Description of Artificial Sequence:peptidic
      inhibitor

<400> 95
Arg Asp Lys Arg Glu Gln

1                   5

<210> 96
<211> 6
<212> PRT
<213> Artificial Sequence

<220>
<223> Description of Artificial Sequence:peptidic
      inhibitor

<400> 96
Arg Asp Lys Arg Lys Lys
  1               5

<210> 97
<211> 6
<212> PRT
<213> Artificial Sequence

<220>
<223> Description of Artificial Sequence:peptidic
      inhibitor

<400> 97
Arg Asp Lys Arg Lys Thr
  1               5

<210> 98
<211> 6
<212> PRT
<213> Artificial Sequence

<220>
<223> Description of Artificial Sequence:peptidic
      inhibitor

<400> 98
Arg Asp Lys Arg Gln Lys
  1               5

<210> 99
<211> 6
<212> PRT
<213> Artificial Sequence

<220>
<223> Description of Artificial Sequence:peptidic
      inhibitor

<400> 99
Arg Asp Lys Arg Gln Asn
  1               5

<210> 100
<211> 6
<212> PRT
<213> Artificial Sequence

<220>
<223> Description of Artificial Sequence:peptidic
      inhibitor

<400> 100
Arg Asp Lys Arg Arg Asn
  1               5

<210> 101
<211> 6
<212> PRT
<213> Artificial Sequence

<220>
<223> Description of Artificial Sequence:peptidic
      inhibitor

<400> 101
Arg Asp Lys Ser Lys Lys
  1               5

<210> 102
<211> 6

<212> PRT
<213> Artificial Sequence

<220>
<223> Description of Artificial Sequence:peptidic
      inhibitor

<400> 102
Arg Asp Lys Thr Gln Gln
  1               5

<210> 103
<211> 6
<212> PRT
<213> Artificial Sequence

<220>
<223> Description of Artificial Sequence:peptidic
      inhibitor

<400> 103
Arg Asp Lys Val Glu Arg
  1               5

<210> 104
<211> 6
<212> PRT
<213> Artificial Sequence

<220>
<223> Description of Artificial Sequence:peptidic
      inhibitor

<400> 104
Arg Asp Lys Val His Lys
  1               5

<210> 105
<211> 6
<212> PRT
<213> Artificial Sequence

<220>
<223> Description of Artificial Sequence:peptidic
      inhibitor

<400> 105
Arg Asp Arg Lys Gln Thr
  1               5

<210> 106
<211> 6
<212> PRT
<213> Artificial Sequence

<220>
<223> Description of Artificial Sequence:peptidic
      inhibitor

<400> 106
Arg Asp Arg Lys Arg Lys
  1               5

<210> 107
<211> 6
<212> PRT
<213> Artificial Sequence

<220>
<223> Description of Artificial Sequence:peptidic
      inhibitor

<400> 107
Arg Asp Arg Gln Lys Lys
  1               5

<210> 108
<211> 6
<212> PRT
<213> Artificial Sequence

<220>
<223> Description of Artificial Sequence:peptidic
      inhibitor

<400> 108
Arg Gly Lys Val Gln Lys
1               5

<210> 109
<211> 6
<212> PRT
<213> Artificial Sequence

<220>
<223> Description of Artificial Sequence:peptidic
      inhibitor

<400> 109
Arg Asn Lys Met Gln Lys
1               5

<210> 110
<211> 6
<212> PRT
<213> Artificial Sequence

<220>
<223> Description of Artificial Sequence:peptidic
      inhibitor

<400> 110
Arg Asn Lys Gln Gln Lys
1               5

<210> 111
<211> 6
<212> PRT
<213> Artificial Sequence

<220>
<223> Description of Artificial Sequence:peptidic
      inhibitor

<400> 111
Arg Asn Lys Arg Lys Thr

1               5

<210> 112
<211> 6
<212> PRT
<213> Artificial Sequence

<220>
<223> Description of Artificial Sequence:peptidic
      inhibitor

<400> 112
Arg Asn Lys Val Gln Lys
  1               5

<210> 113
<211> 6
<212> PRT
<213> Artificial Sequence

<220>
<223> Description of Artificial Sequence:peptidic
      inhibitor

<400> 113
Arg Asn Lys Val Arg Lys
  1               5

<210> 114
<211> 6
<212> PRT
<213> Artificial Sequence

<220>
<223> Description of Artificial Sequence:peptidic
      inhibitor

<400> 114
Arg Asn Gln Val Arg Lys
  1               5

<210> 115
<211> 6
<212> PRT
<213> Artificial Sequence

<220>
<223> Description of Artificial Sequence:peptidic
      inhibitor

<400> 115
Ser Asp Lys Lys Lys Gln
  1               5

<210> 116
<211> 6
<212> PRT
<213> Artificial Sequence

<220>
<223> Description of Artificial Sequence:peptidic
      inhibitor

<400> 116
Ser Asp Lys Lys Gln Lys
  1               5

<210> 117
<211> 6
<212> PRT
<213> Artificial Sequence

<220>
<223> Description of Artificial Sequence:peptidic
      inhibitor

<400> 117
Thr Asp Lys Lys Lys Gln
  1               5

<210> 118
<211> 6

<212> PRT
<213> Artificial Sequence

<220>
<223> Description of Artificial Sequence:peptidic
      inhibitor

<400> 118
Thr Asp Lys Lys Gln Lys
  1               5

<210> 119
<211> 6
<212> PRT
<213> Artificial Sequence

<220>
<223> Description of Artificial Sequence:peptidic
      inhibitor

<400> 119
Thr Asp Lys Arg Lys Gln
  1               5

<210> 120
<211> 6
<212> PRT
<213> Artificial Sequence

<220>
<223> Description of Artificial Sequence:peptidic
      inhibitor

<400> 120
Val Asp Lys Gln Gln Gln
  1               5

<210> 121
<211> 6
<212> PRT
<213> Artificial Sequence

<220>
<223> Description of Artificial Sequence:peptidic
      inhibitor

<400> 121
Arg Asp Lys Val Lys Ala
  1               5

<210> 122
<211> 6
<212> PRT
<213> Artificial Sequence

<220>
<223> Description of Artificial Sequence:peptidic
      inhibitor

<400> 122
Arg Asp Ala Val Lys Lys
  1               5

<210> 123
<211> 6
<212> PRT
<213> Artificial Sequence

<220>
<223> Description of Artificial Sequence:peptidic
      inhibitor

<400> 123
Arg Asp Lys Val Ala Lys
  1               5

<210> 124
<211> 6
<212> PRT
<213> Artificial Sequence

<220>
<223> Description of Artificial Sequence:peptidic
      inhibitor

<400> 124
Arg Asp Lys Val Ala Ala
  1               5

<210> 125
<211> 6
<212> PRT
<213> Artificial Sequence

<220>
<223> Description of Artificial Sequence:peptidic
      inhibitor

<400> 125
Arg Asp Ala Val Lys Ala
  1               5

<210> 126
<211> 6
<212> PRT
<213> Artificial Sequence

<220>
<223> Description of Artificial Sequence:peptidic
      inhibitor

<400> 126
Arg Asp Ala Val Ala Lys
  1               5

<210> 127
<211> 15
<212> PRT
<213> Artificial Sequence

<220>
<223> Description of Artificial Sequence:peptidic
      inhibitor

<400> 127
Cys Ser Phe Asn Ile Thr Thr Glu Ile Arg Asp Lys Val Lys Lys

1                5                   10                  15

<210> 128
<211> 21
<212> DNA
<213> Artificial Sequence

<220>
<223> Description of Artificial Sequence:primer

<400> 128
ggctaactag ggaacccact g                                                21

<210> 129
<211> 24
<212> DNA
<213> Artificial Sequence

<220>
<223> Description of Artificial Sequence:primer

<400> 129
ctgctagaga ttttccacac tgac                                             24

<210> 130
<211> 21
<212> DNA
<213> Artificial Sequence

<220>
<223> Description of Artificial Sequence:primer

<400> 130
tgtgtgcccg tctgttgtgt g                                                21

<210> 131
<211> 20
<212> DNA
<213> Artificial Sequence

<220>
<223> Description of Artificial Sequence:primer

<400> 131
accacagtcc atgccatcac                                                    20

<210> 132
<211> 21
<212> DNA
<213> Artificial Sequence

<220>
<223> Description of Artificial Sequence:primer

<400> 132
tccaccaccc tgttgctgta g                                                  21